# EUROPÄISCHE PATENTSCHRIFT

(11) **EP 0 810 999 B1**
(45) Veröffentlichungstag und Bekanntmachung des Hinweises auf die Patenterteilung: **12.01.2000**
(21) Anmeldenummer: 96904760.4
(22) Anmeldetag: 13.02.1996
(51) Int. Cl.: C07D 261/08, C07D 417/10, C07D 413/10, A01N 43/80

(54) **ISOXAZOLYL-BENZOYLDERIVATE**
ISOXAZOLYL-BENZOYL DERIVATIVES
DERIVES D'ISOXAZOLYLBENZOYLE

(30) Priorität: 24.02.1995 DE 19506573
(43) Veröffentlichungstag der Anmeldung: 10.12.1997
(73) Patentinhaber: BASF AKTIENGESELLSCHAFT, 67056 Ludwigshafen (DE)
(72) Erfinder: VON DEYN, Wolfgang, D-67434 Neustadt (DE); HILL, Regina, Luise, D-67346 Speyer (DE); KARDORFF, Uwe, D-68159 Mannheim (DE); ENGEL, Stefan, D-65510 Idstein (DE); OTTEN, Martina, D-67069 Ludwigshafen (DE); VOSSEN, Marcus, D-68199 Mannheim (DE); PLATH, Peter, D-67227 Frankenthal (DE); RANG, Harald, D-67122 Altrip (DE); HARREUS, Albrecht, D-67063 Ludwigshafen (DE); RÖHL, Franz, D-67105 Schifferstadt (DE); WALTER, Helmut, D-67283 Obrigheim (DE); WESTPHALEN, Karl-Otto, D-67346 Speyer (DE); MISSLITZ, Ulf, D-67433 Neustadt (DE)
(86) Internationale Anmeldenummer: EP9600592
(87) Internationale Veröffentlichungsnummer: WO9626192

(56) Entgegenhaltungen:
- EP-A- 0 418 175
- EP-A- 0 609 797

## Beschreibung

Die vorliegende Erfindung betrifft neue Isoxazolyl-benzoylderivate mit herbizider Wirkung, Verfahren zur Herstellung der Isoxazolyl-benzoylderivate, Mittel welche diese enthalten sowie die Verwendung dieser Derivate oder sie enthaltender Mittel zur Unkrautbekämpfung.

Aus der Literatur sind herbizidwirksame Isoxazolyl-benzoylderivate bekannt, beispielsweise aus EP 609797.

In der europäischen Patentanmeldung EP-A-0 418 175 werden 4-Benzoylisoxazolderivate mit herbiziden Eigenschaften offenbart. Die Benzoylgruppe ist nicht durch eine heterocyclische Gruppe substituiert.

Die herbiziden Eigenschaften der bekannten Verbindungen sowie die Verträglichkeit gegenüber Kulturpflanzen können jedoch nur bedingt befriedigen.

Die Aufgabe bestand darin neue Isoxazolyl-benzoylderivate mit verbesserten Eigenschaften zu finden.

Es wurden nun neue Isoxazolyl-benzoylderivate der Formel I gefunden 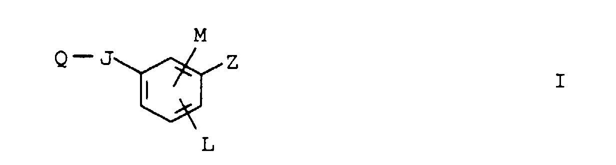 in der die Substituenten folgende Bedeutung haben:
- L, M: Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können, Halogen, Cyano, Nitro, eine Gruppe -(Y)ₙ-S(O)ₘR⁷ oder eine Gruppe -(Y)ₙ-CO-R⁸
- z: ein 5- oder 6-gliedriger heterocyclischer, gesättigter oder ungesättigter Rest, enthaltend ein bis drei Heteroatome, ausgewählt aus der Gruppe Sauerstoff, Schwefel oder Stickstoff, der gegebenenfalls durch Halogen, Cyano, Nitro, eine Gruppe -CO-R⁸, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl oder eine Oxogruppe, die gegebenenfalls auch in der tautomeren Form als Hydroxygruppe vorliegen kann, substituiert ist oder der mit einem ankondensierten, gegebenenfalls durch Halogen, Cyano, Nitro, C₁-C₄-Alkyl oder C₁-C₄-Halogenalkyl substituierten Phenylring, einem ankondensierten Carbocyclus oder einem ankondensierten, gegebenenfalls durch Halogen, Cyano, Nitro, C₁-C₄-Alkyl, Di-C₁-C₄-Alkylamino, C₁-C₄-Alkoxy, C₁-C₄-Halogenalkoxy, oder C₁-C₄-Halogenalkyl substituierten zweiten Heterocyclus ein bicyclisches System bildet.

- Y: O, NR⁹
- n: null oder eins
- m: null, eins oder zwei
- R⁷: C₁-C₄-Alkyl, C₁-C₄-Halogenalkyl oder NR⁹R¹⁰
- R⁸: C₁-C₄-Alkyl, C₁-C₄-Halogenalkyl, C₁-C₄-Alkoxy, oder NR⁹R¹⁰
- R⁹: Wasserstoff oder C₁-C₄-Alkyl
- R¹⁰: C₁-C₄-Alkyl
- Q: ein in Stellung 4 verknüpfter Isoxazolring der Formel II 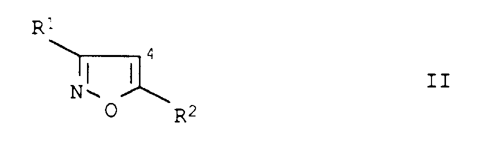
in welcher
- R¹: für Wasserstoff, C₁-C₄-Alkyl oder -CO₂R³
- R²: für Wasserstoff, C₁-C₄-Alkyl oder C₃-C₆-Cycloalkyl, welches gegebenenfalls durch ein oder mehrere Halogenatome oder C₁-C₄-Alkyl substituiert sein kann und
- R³: für C₁-C₄-Alkyl steht;
- J: ein Carbonylrest CO oder ein Rest -CHR⁴;
- R⁴: eine OH-Gruppe, eine ggf. mit C₂-C₄-Acyl acylierte Hydroxylgruppe oder ein Chloratom bedeutet;
sowie landwirtschaftlich übliche Salze der Verbindungen I.

Verbindungen der Formel I mit R¹ = H erhält man dadurch, daß man die an sich bekannten β-Ketoester der Formel A1 [Y. Oikawa et al., JOC 43, 2087 (1978)] mit einem Benzoesäurederivat der Formel III (T = Cl) zu einem Ester der Formel B1 acyliert, dieses durch Umsetzung mit p-Toluolsulfonsäure in Toluol in ein 1,3-Diketon der Formel Cl überführt, danach das 1,3-Diketon C1 entweder mit Orthoameisensäuretriethylester in einen Enolether D1 oder mit Dimethylformamid-dimethylacetal in ein Enamin D2 überführt und D1 oder D2 mit Hydroxylamin zum Isoxazolyl-benzoylderivat der Formel I.d umsetzt. Die Reaktionssequenz ist im nachfolgenden Formelschema zusammengefaßt:

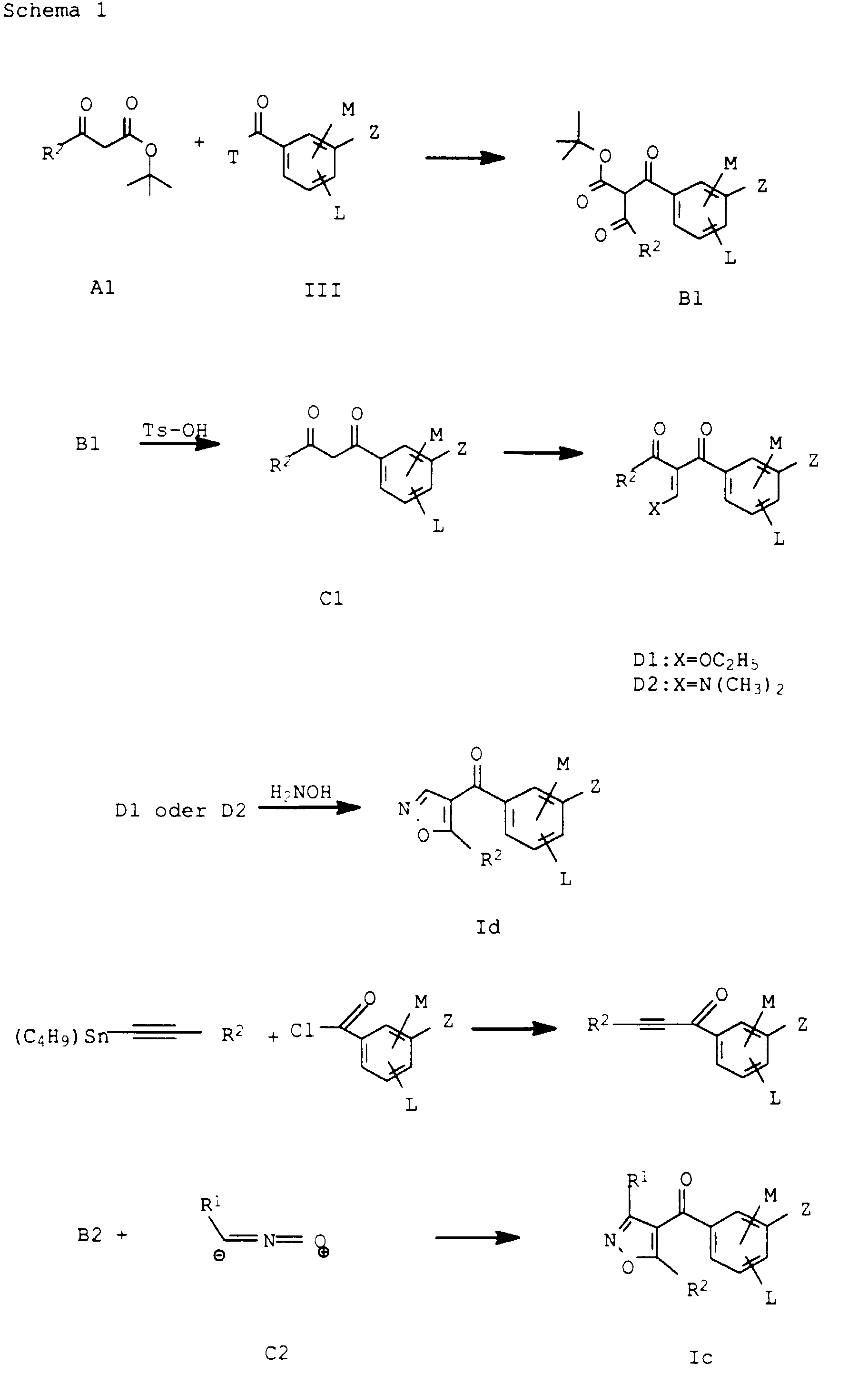

Benzoesäurederivate der Formel III lassen sich folgendermaßen herstellen:

Benzoylhalogenide wie beispielsweise Benzoylchloride der Formel III (T = Cl) werden in an sich bekannter Weise durch Umsetzung der Benzoesäuren der Formel III (T = OH) mit Thionylchlorid hergestellt.

Die Benzoesäuren der Formel III (T = OH) können in bekannter Weise durch saure oder basische Hydrolyse aus den entsprechenden Estern der Formel III (T = C₁-C₄-Alkoxy) hergestellt werden.

Die Zwischenprodukte der Formel III lassen sich z.B. gemäß Schema 2 und 3 auf den im folgenden beschriebenen Wegen darstellen. 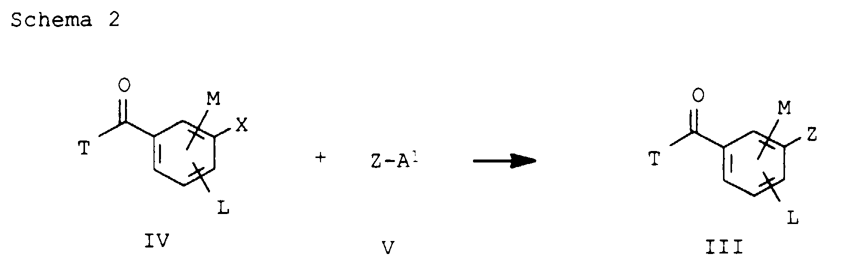
- T: C₁-C₄-Alkoxy,
- x: Cl, Br, J, -OS(O)₂CF₃, -OS(O)₂F
- A¹: Sn( C₁-C₄-Alkyl)₃, B(OH)₂, ZnHal, wobei Hal für Cl oder Br steht
- L, M, Z: wie oben definiert.

Danach lassen sich die Arylhalogenverbindungen oder Arylsulfonate IV in an sich bekannter Weise mit Heteroarylstannaten (Stille-Kupplungen), Heteroaryl-Borverbindungen (Suzuki-Kupplungen) oder Heteroaryl-Zinkverbindungen (Negishi-Reaktion) V (vgl. z.B. Synthesis 1987, 51-53, Synthesis 1992, 413) in Gegenwart eines Palladium- oder Nickel-Übergangsmetallkatalysators und gegebenenfalls einer Base zu den neuen Verbindungen der allgemeinen Formel III umsetzen.

Die Benzoesäurederivate der Formel III können auch erhalten werden, indem man entsprechende brom- oder iodsubstituierte Verbindungen der Formel VI 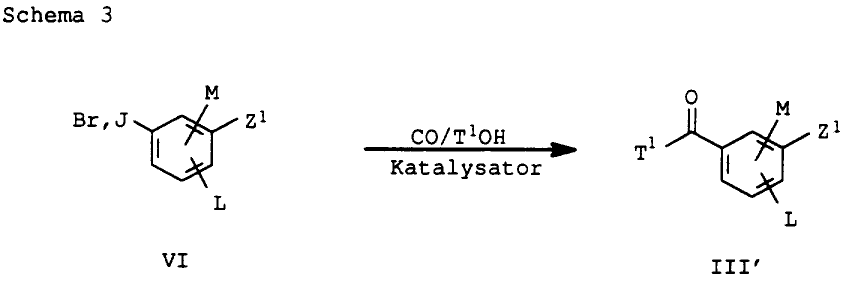
- Z¹: Z oder CN
- T¹: OH, C₁-C₄-Alkoxy
in der L und M die obengenannte Bedeutung haben, in Gegenwart eines Palladium-, Nickel-, Cobalt- oder Rhodium-Übergangsmetallkatalysators und einer Base mit Kohlenmonoxid und Wasser unter erhöhtem Druck umsetzt.

Bevorzugt im Rahmen der vorliegenden Erfindung sind Isoxazol-4-ylbenzoylderivate der Formel Ia 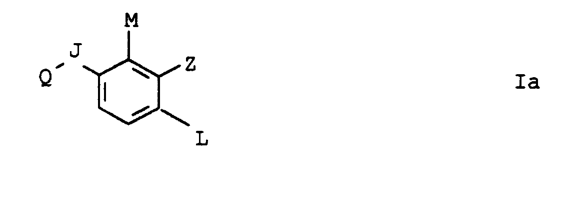 in der
- L: für C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsuflonyl, Halogen, Nitro oder Cyano und
- M: für Wasserstoff, C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano steht und Q, J und
- Z: die oben angegebenen Bedeutungen haben.

Bevorzugt sind auch Isoxazol-4-yl-benzoylderivate der Formel Ib 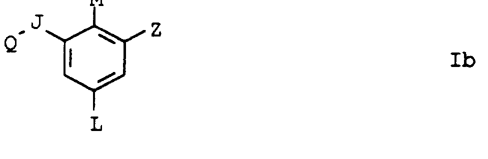 in der
- L und M: für C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano stehen und Q, J und
- Z: die oben angegebenen Bedeutungen haben.

Die Katalysatoren Nickel, Cobalt, Rhodium und insbesondere Palladium können metallisch oder in Form üblicher Salze wie in Form von Halogenverbindungen, z.B. PdCl₂, RhCl₃·H₂O, Acetaten, z.B. Pd(OAc)₂, Cyaniden usw. in den bekannten Wertigkeitsstufen vorliegen. Ferner können Metallkomplexe mit tertiären Phosphinen, Metallalkylcarbonyle, Metallcarbonyle, z.B. CO₂(CO)₈, Ni(CO)₄, Metallcarbonyl-Komplexe mit tertiären Phosphinen, z.B. (PPh₃)₂Ni(CO)₂, oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 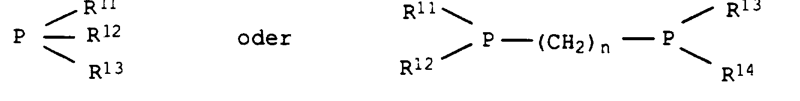 wobei n die Zahlen 1, 2, 3 oder 4 bedeutet und die Reste R¹¹ bis R¹⁴ für niedermolekulares Alkyl, z.B. C₁-C₆-Alkyl, Aryl, C₁-C₄-Alkylaryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise, z.B. wie in den eingangs genannten Dokumenten beschrieben, erfolgen. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)₂, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Moläquivalente, besonders bevorzugt 1 bis 5 Moläquivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff II bzw. III verwenden.

Zur Herstellung der Benzoesäuren III (T = OH) führt man die Umsetzung mit Kohlenmonoxid und mindestens äquimolaren Mengen an Wasser, bezogen auf die Ausgangsstoffe VI durch. Der Reaktionspartner Wasser kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether z.B. Methyl-tert.butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base, im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie tert.-Alkylamine, z.B. Trialkylamine wie Triethylamin, cyclische Amine wie N-Methylpiperidin oder N,N'-Dimethylpiperazin, Pyridin, Alkali- oder -hydrogencarbonate, oder tetraalkylsubstituierte Harnstoffderivate wie Tetra-C₁-C₄-alkylharnstoff, z.B. Tetramethylharnstoff, zu nennen.

Die Menge an Base ist nicht kritisch, üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf VI vorliegt. Vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Die als Ausgangsverbindungen benutzten Arylhalogenverbindungen VI sind bekannt oder können leicht durch geeignete Kombination bekannter Synthesen hergestellt werden.

Beispielsweise können die Halogenverbindungen VI durch Sandmeyer-Reaktion aus entsprechenden Anilinen erhalten werden, die ihrerseits durch Reduktion von geeigneten Nitroverbindungen (vgl. z.B. für VI mit Z¹ = CN: Liebigs Ann. Chem. 1980, 768-778) synthetisiert werden. Die Arylbromide VI können außerdem durch direkte Bromierung geeigneter Ausgangsverbindungen erhalten werden [vgl. z.B. Monatsh. Chem. 99, 815-822 (1968)]. 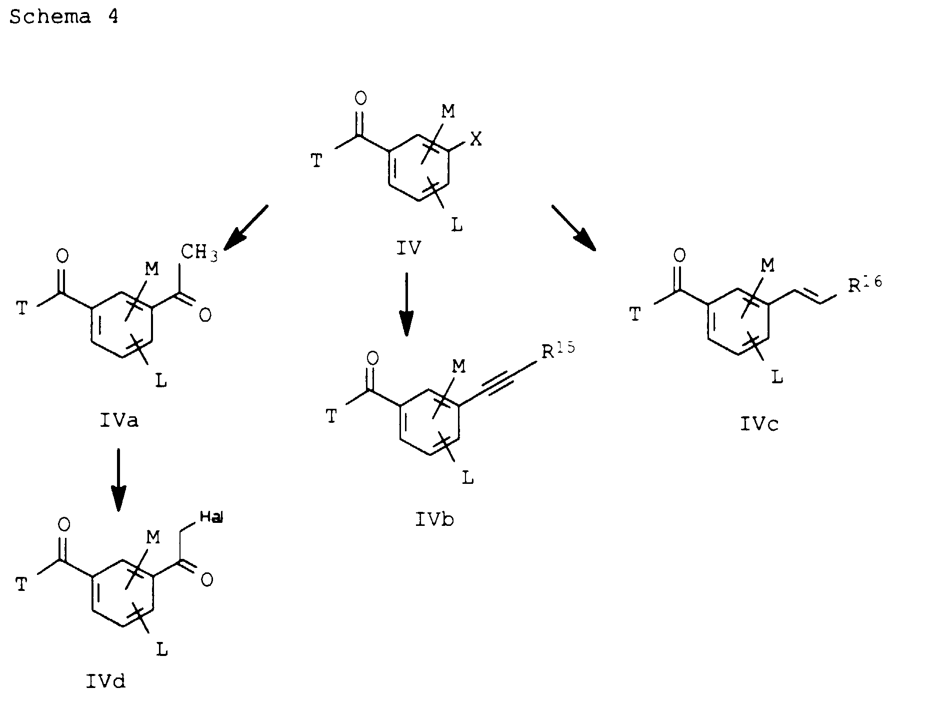
- T: C₁-C₄-Alkoxy
- X: Cl, Br, J, -OS(O)₂CF₃, -OS(O)₂F
- L,M,Z: wie oben definiert
- R¹⁵: Wasserstoff, C₁-C₄-Alkyl, C₁-C₄-Halogenalkyl, C₃-C₈-Cycloalkyl, ggf. subst. Phenyl oder Trimethylsilyl,
- R¹⁶: Wasserstoff, C₁-C₄-Halogenalkyl, C₃-C₈-Cycloalkyl oder ggf. subst. Phenyl.

Ausgehend von den Arylhalogenverbindungen oder Arylsulfonaten IV lassen sich in Gegenwart eines Palladium- oder Nickel-Übergangsmetallkatalysators und gegebenenfalls einer Base Arylmethylketone IVa nach literaturbekannten Verfahren durch Umsetzung mit Vinylalkylethern und anschließende Hydrolyse herstellen [vgl. z.B. Tetrahedron Lett. 32, 1753-1756 (1991)].

Die ethinylierten Aromaten IVb können in an sich bekannter Weise durch Umsetzung von Arylhalogenverbindungen oder Arylsulfonaten IV mit substituierten Acetylenen in Gegenwart eines Palladium- oder Nickel-Übergangsmetallkatalysators hergestellt werden (z.B. Heterocycles, 24, 31-32 (1986)). Derivate IVb mit R¹⁵= H erhält man zweckmäßigerweise aus den Silylverbindungen IVb, R¹⁵= -Si(CH₃)₃ [J.Org.Chem. 46, 2280-2286 (1981)].

Durch Heck-Reaktion von Arylhalogenverbindungen oder Arylsulfonaten IV mit Olefinen in Gegenwart eines Palladiumkatalysators werden die Arylalkene IVc erhalten (vgl. z.B. Heck, Palladium Reagents in Organic Synthesis, Academic Pres, London 1985 bzw. Synthesis 1993, 735-762).

Die als Ausgangsverbindungen benutzten Benzoylderivate IV sind bekannt [ vgl. z.B.Coll. Czech. Chem. Commn. 40, 3009-3019 (1975)] oder können leicht durch geeignete Kombination bekannter Synthesen hergestellt werden.

Beispielsweise können die Sulfonate IV (X = -OS(O)₂CF₃, -OS(O)₂F) aus den entsprechenden Phenolen, die ihrerseits bekannt sind (vgl. z.B. EP 195247) oder nach bekannten Methoden hergestellt werden können, erhalten werden ( vgl. z.B. Synthesis 1993, 735-762).

Die Halogenverbindungen IV (X = Cl, Br oder I) können beispielsweise durch Sandmeyer-Reaktion aus entsprechenden Anilinen erhalten werden. 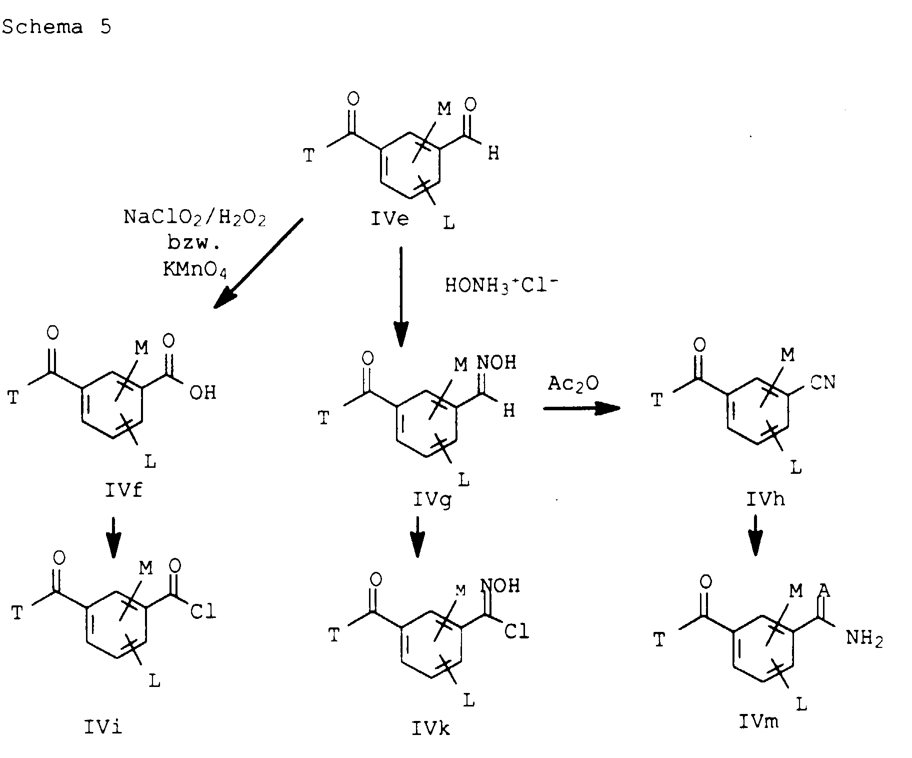
- A: S, NH oder NOH
- T,: ist C₁-C₄-Alkoxy und L, M wie oben definiert.

Isophthalsäurederivate IVf können aus den Aldehyden IVe nach bekannten Verfahren hergestellt werden [ s. J. March Advanced Organic Chemistry 3. Aufl., S. 629ff, Wiley-Interscience Publication (1985)].

Die Oxime IVg erhält man vorteilhaft dadurch, daß man in an sich bekannter Weise Aldehyde IVe mit Hydroxylamin umsetzt [ s. J. March Advanced Organic Chemistry 3. Aufl., S. 805-806, Wiley-Interscience Publication (1985)].

Die Umwandlung der Oxime IVg in Nitrile IVh kann ebenfalls nach an sich bekannten Verfahren erfolgen [s. J. March Advanced Organic Chemistry 3. Aufl., S. 931-932, Wiley-Interscience Publication (1985)].

Die als Ausgangsverbindungen benötigten Aldehyde IVe sind bekannt oder nach bekannten Methoden herstellbar. Beispielsweise können sie gemäß Schema 6 aus den Methylverbindungen VII synthetisiert werden.

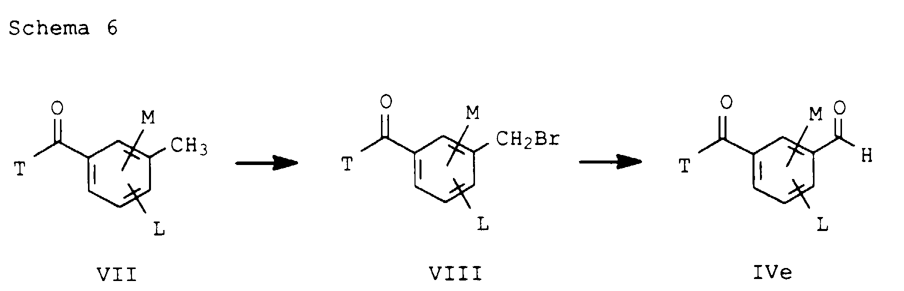

Die Reste T, M und L haben die unter Schema 5 genannte Bedeutung. Die Methylverbindungen VII können nach allgemein bekannten Methoden, beispielsweise mit N-Bromsuccinimid oder 1,3-Dibrom-5,5-dimethylhydantoin, zu den Benzylbromiden VIII umgesetzt werden. Die Umsetzung von Benzylbromiden zu Benzaldehyden IVe ist ebenfalls literaturbekannt [vgl. Synth. Commun. 22 1967-1971 (1992)].

Die Vorprodukte IVa bis IVh eignen sich zum Aufbau heterocyclischer Zwischenprodukte III.

Beispielsweise können aus den Acetophenonen IVa über die halogenierte Zwischenstufe IVd 5-Oxazolyl-[ vgl. z.B. J. Heterocyclic Chem., 28, 17-28 (1991)] oder 4-Thiazolyl-derivate [vgl. z.B. Metzger, Thiazoles *in*: The Chemistry of heterocyclic compounds, Vol.34 S. 175ff (1976)] erhalten werden.

Die Acetylene IVb bzw. die Alkene IVc eignen sich zum Aufbau von 4-Isoxazolyl-, 5-Isoxazolyl-, 4,5-Dihydroisoxazol-4-yl-, 4,5-Dihydroisoxazol-5-yl-derivaten [vgl. z.B. Houben-Weyl, Methoden der organischen Chemie, 4. Aufl., Bd. X/3, S. 843ff (1965)].

Aus den Benzoesäuren IVf bzw. den daraus nach Standardverfahren erhältlichen Säurechloriden IVi können beispielsweise nach literaturbekannten Verfahren 2-Oxazolyl-, 1,2,4-Oxadiazol-5-yl-, 1,3,4-Oxadiazol-2-yl-derivate [ vgl. z.B. J. Heterocyclic Chem., 28, 17-28 (1991)] oder 2-Pyrrolyl-derivate [ vgl. z.B. Heterocycles 26, 3141-3151 (1987)] hergestellt werden.

1,2,4-Triazol-3-yl-derivate sind aus Benzonitrilen IVh nach bekannten Methoden [vgl. z.B. J. Chem. Soc. 3461-3464 (1954)] herzustellen.

Die Benzonitrile IVh können über die Zwischenstufe der Thioamide, Amidoxime oder Amdine IVm in 1,2,4-Oxadiazol-3-yl- [ vgl. z.B. J. Heterocyclic Chem., 28, 17-28 (1991)] 2-Thiazolyl-, 4,5-Dihydrothiazol-2-yl- oder 5,6-Dihydro-4-H-1,3-thiazin-2-yl-derivate [vgl. z.B. Houben-Weyl, Methoden der organischen Chemie, 4. Aufl., Bd. E5, S. 1268ff (1985)] umgewandelt werden. Aus den Thioamiden IVm (A=S) sind nach literaturbekannten Verfahren auch 1,2,4-Thiadiazol-5-yl-derivate [ vgl. z.B. J.Org.Chem. 45 3750-3753 (1980)] oder 1,3,4-Thiadiazol-2-yl-derivate [ vgl. z.B. J. Chem.Soc., Perkin Trans. I 1987-1991 (1982)] erhältlich.

Die Umwandlung von Oximen IVg in 3-Isoxazolyl-derivate kann in an sich bekannter Weise über die Zwischenstufe der Hydroxamsäurechloride IVk erfolgen [vgl. z.B. Houben-Weyl, Methoden der organischen Chemie, 4. Aufl., Bd. X/3, S. 843ff (1965)].

Die Verbindungen I mit J = -CHR⁴ können aus den Verbindungen der Formel Ib mit J = CO dadurch erhalten werden, daß man sie mit Natriumborhydrid zu Carbinolen der Formel I.3 reduziert, und diese entweder mit Methansulfonsäurechlorid zu Chloriden der Formel I.4 umsetzt oder mit C₂-C₄-Carbonsäureanhydriden zu Estern der Formel I.5 acyliert.

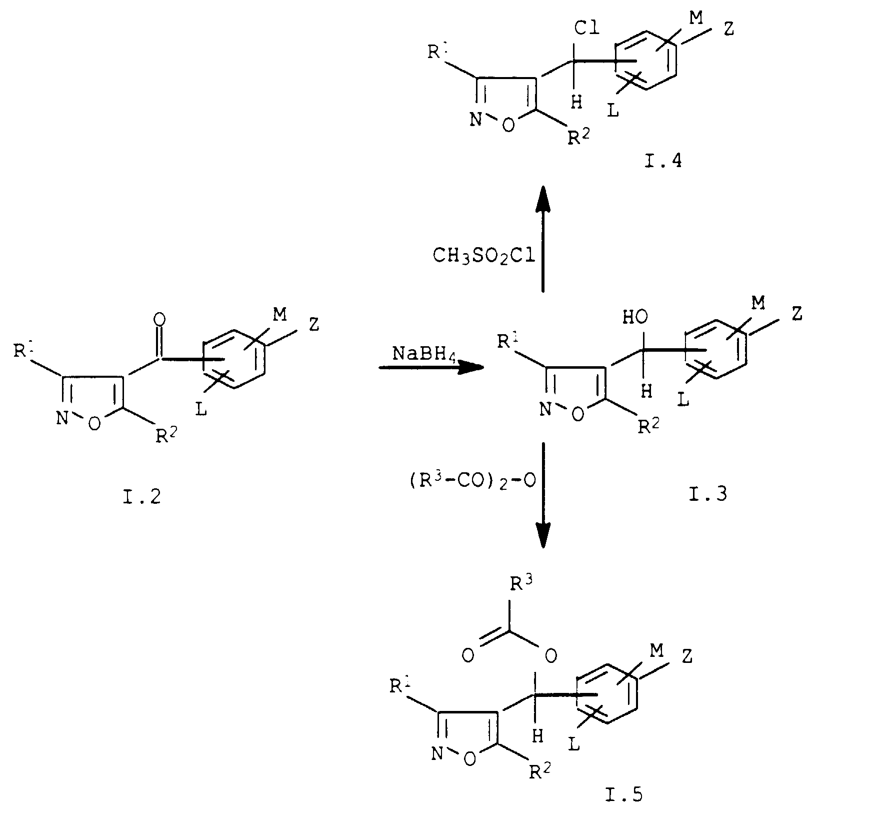

Im Hinblick auf die bestimmungsgemäße Verwendung der Isoxazol-4-yl-benzoylderivate der allgemeinen Formel I kommen als Substituenten folgende Reste in Betracht:
L,M Wasserstoff,
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-methylpropyl oder 1-Ethyl-2-methyl-propyl,
insbesondere Methyl, Ethyl, 1-Methylethyl, 1-Methylpropyl, 2-Methylpropyl, 1,1-Dimethylethyl und 1,1-Dimethylpropyl;
C₂-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3 butenyl, 1,1-Dimethyl-2-propenyl,1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3 pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3 butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
insbesondere 1-Methyl-2-propenyl, 1-Methyl-2-butenyl, 1,1-Dimethyl-2-propenyl und 1,1-Dimethyl-2-butenyl;
C₂-C₆-Alkinyl wie Propargyl, 2-Butinyl, 3-Butenyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2 propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy,
insbesondere C₁-C₃-Alkoxy wie Methoxy, Ethoxy, i-Propoxy,
wobei diese Gruppen gegebenenfalls durch ein bis fünf Halogenatome wie Fluor, Chlor, Brom und Iod, vorzugsweise Fluor und Chlor oder C₁-C₄-Alkoxy wie vorstehend genannt substituiert sein können.

Die vorstehend definierte Gruppe -(Y)ₙ-S(O)ₘR⁷ steht beispielsweise für
C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio;
C₁-C₄-Alkylsulfinyl wie Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl und 1,1-Dimethylethylsulfinyl, insbesondere Methylsulfinyl;
C₁-C₄-Alkylsulfonyl wie Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl und 1,1-Dimethylethylsulfonyl, insbesondere Methylsulfonyl;
C₁-C₄-Alkoxysulfonyl wie Methoxysulfonyl, Ethoxysulfonyl, n-Propoxysulfonyl, 1-Methylethoxysulfonyl, n-Butoxysulfonyl, 1-Methylpropoxysulfonyl, 2-Methylpropoxysulfonyl und 1,1-Dimethylethoxysulfonyl, insbesondere Methoxysulfonyl;
N-C₁-C₄-Alkylsulfamoyl wie N-Methylsulfamoyl, N-Ethylsulfamoyl, N-n-Propylsulfamoyl, N-1-Methylethylsulfamoyl, N-n-Butylsulfamoyl, N-1-Methylpropylsulfamoyl, N-2-Methylpropylsulfamoyl und N-1,1-Dimethylethylsulfamoyl, insbesondere N-Methylsulfamoyl;
N-C₁-C₄-Alkylsulfinamoyl wie N-Methylsulfinamoyl, N-Ethylsulfinamoyl, N-n-Propylsulfinamoyl, N-1-Methylethylsulfinamoyl, N-n-Butylsulfinamoyl, N-1-Methylpropylsulfinamoyl, N-2-Methylpropylsulfinamoyl und N-1,1-Dimethylethylsulfinamoyl, insbesondere N-Methylsulfinamoyl;
Di-C₁-C₄-Alkylsulfamoyl wie Dimethylsulfamoyl, Diethylsulfamoyl, Dipropylsulfamoyl, Dibutylsulfamoyl, N-Methyl-N-ethylsulfamoyl, N-Methyl-N-propylsulfamoyl, N-Methyl-N-1-methylethylsulfamoyl, N-Methyl-N-1,1-Dimethylethylsulfamoyl, Di-1-Methylethylsulfamoyl, N-Ethyl-N-1-Methylethylsulfamoyl und N-Ethyl-N-1,1-dimethylethylsulfamoyl; insbesondere Dimethylsulfamoyl;
Di-C₁-C₄-Alkylsulfinamoyl wie Dimethylsulfinamoyl, Diethylsulfinamoyl, Dipropylsulfinamoyl, Dibutylsulfinamoyl, N-Methyl-N-ethylsulfinamoyl, N-Methyl-N-propylsulfinamoyl, N-Methyl-N-1-methylethylsulfinamoyl, N-Methyl-N-1,1-Dimethylethylsulfinamoyl, Di-1-Methylethylsulfinamoyl, N-Ethyl-N-1-Methylethylsulfinamoyl und N-Ethyl-N-1,1-dimethylethylsulfinamoyl; insbesondere Dimethylsulfinamoyl,
C₁-C₄-Alkylsulfinyloxy wie Methylsulfinyloxy, Ethylsulfinyloxy, n-Propylsulfinyloxy, 1-Methylethylsulfinyloxy, n-Butylsulfinyloxy, 1-Methylpropylsulfinyloxy, 2-Methylpropylsulfinyloxy und 1,1-Dimethylethylsulfinyloxy, insbesondere Methylsulfinyloxy;
C₁-C₄-Alkylsulfonyloxy wie Methylsulfonyloxy, Ethylsulfonyloxy, n-Propylsulfonyloxy, 1-Methylethylsulfonyloxy, n-Butylsulfonyloxy, 1-Methylpropylsulfonyloxy, 2-Methylpropylsulfonyloxy und 1,1-Dimethylethylsulfonyloxy, insbesondere Methylsulfonyloxy;
C₁-C₄-Alkylsulfinylamino wie Methylsulfinylamino, Ethylsulfinylamino, n-Propylsulfinylamino, 1-Methylethylsulfinylamino, n-Butylsulfinylamino, 1-Methylpropylsulfinylamino, 2-Methylpropylsulfinylamino und 1,1-Dimethylethylsulfinylamino, insbesondere Methylsulfinylamino;
C₁-C₄-Alkylsulfonylamino wie Methylsulfonylamino, Ethylsulfonylamino, n-Propylsulfonylamino, 1-Methylethylsulfonylamino, n-Butylsulfonylamino, 1-Methylpropylsulfonylamino, 2-Methylpropylsulfonylamino und 1,1-Dimethylethylsulfonylamino, insbesondere Methylsulfonylamino;
N-C₁-C₄-Alkylsulfinyl-N-methyl-amino wie N-Methylsulfinyl-N-methyl-amino, N-Ethylsulfinyl-N-methyl-amino, N-n-Propylsulfinyl-N-methyl-amino, N-1-Methylethylsulfinyl-N-methyl-amino, N-n-Butylsulfinyl-N-methyl-amino, N-1-Methylpropylsulfinyl-N-methyl-amino, N-2-Methylpropylsulfinyl-N-methyl-amino und N-1,1-Dimethylethylsulfinyl-N-methyl-amino, insbesondere N-Methylsulfinyl-N-methylamino;
N-C₁-C₄-Alkylsulfinyl-N-ethyl-amino wie N-Methylsulfinyl-N-ethyl-amino, N-Ethylsulfinyl-N-ethyl-amino, N-n-Propylsulfinyl-N-ethylamino, N-1-Methylethylsulfinyl-N-ethyl-amino, N-n-ButylsulfinylN-ethyl-amino, N-1-Methylpropylsulfinyl-N-ethyl-amino, N-2-Methylpropylsulfinyl-N-ethyl-amino und N-1,1-Dimethylethylsulfinyl-N-ethyl-amino, insbesondere N-Methylsulfinyl-N-ethyl-amino;
N-C₁-C₄-Alkylsulfonyl-N-methyl-amino wie N-Methylsulfonyl-N-methyl-amino, N-Ethylsulfonyl-N-methyl-amino, N-n-Propylsulfonyl-N-methyl-amino, N-1-Methylethylsulfonyl-N-methyl-amino, N-n-Butylsulfonyl-N-methyl-amino, N-1-Methylpropylsulfonyl-N-methyl-amino, N-2-Methylpropylsulfonyl-N-methyl-amino und N-1,1-Dimethylethylsulfonyl-N-methyl-amino, insbesondere N-Methylsulfonyl-N-methylamino;
N-C₁-C₄-Alkylsulfonyl-N-ethyl-amino wie N-Methylsulfonyl-N-ethyl-amino, N-Ethylsulfonyl-N-ethyl-amino, N-n-Propylsulfonyl-N-ethylamino, N-1-Methylethylsulfonyl-N-ethyl-amino, N-n-Butylsulfonyl-N-ethyl-amino, N-1-Methylpropylsulfonyl-N-ethyl-amino, N-2-Methylpropylsulfonyl-N-ethyl-amino und N-1,1-Dimethylethylsulfonyl-N-ethyl-amino, insbesondere N-Methylsulfonyl-N-ethyl-amino;
C₁-C₄-Halogenalkylthio wie Chlormethylthio, Dichlormethylthio, Trichlormethylthio, Fluormethylthio, Difluormethylthio, Trifluormethylthio, Chlorfluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2 fluorethylthio, 2,2,2-Trichlorethylthio und Pentafluorethylthio, insbesondere Trifluormethylthio.

Die vorstehend definierte Gruppe -(Y)ₙ-CO-R⁸ steht beispielsweise für
C₁-C₄-Alkylcarbonyl wie Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl und 1,1-Dimethylethylcarbonyl, insbesondere Methylcarbonyl;
C₁-C₄-Alkoxycarbonyl wie Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl und 1,1-Dimethylethoxycarbonyl, insbesondere Methoxycarbonyl;
N-C₁-C₄-Alkylcarbamoyl wie N-Methylcarbamoyl, N-Ethylcarbamoyl, N-n-Propylcarbamoyl, N-1-Methylethylcarbamoyl, N-n-Butylcarbamoyl, N-1-Methylpropylcarbamoyl, N-2-Methylpropylcarbamoyl und N-1,1-Dimethylethylcarbamoyl, insbesondere N-Methylcarbamoyl;
Di-C₁-C₄-Alkylcarbamoyl wie Dimethylcarbamoyl, Diethylcarbamoyl, Dipropylcarbamoyl, Dibutylcarbamoyl, N-Methyl-N-ethylcarbamoyl, N-Methyl-N-propylcarbamoyl, N-Methyl-N-1-methylethylcarbamoyl, N-Methyl-N-1,1-Dimethylethylcarbamoyl, Di-1-Methylethylcarbamoyl, N-Ethyl-N-1-Methylethylcarbamoyl und N-Ethyl-N-1,1-dimethyl ethylcarbamoyl; insbesondere Dimethylcarbamoyl;
C₁-C₄-Alkylcarbonyloxy wie Methylcarbonyloxy, Ethylcarbonyloxy, n-Propylcarbonyloxy, 1-Methylethylcarbonyloxy, n-Butylcarbonyloxy, 1-Methylpropylcarbonyloxy, 2-Methylpropylcarbonyloxy und 1,1-Dimethylethylcarbonyloxy, insbesondere Methylcarbonyloxy; C₁-C₄-Alkylcarbonylamino wie Methylcarbonylamino, Ethylcarbonylamino, n-Propylcarbonylamino, 1-Methylethylcarbonylamino, n-Butylcarbonylamino, 1-Methylpropylcarbonylamino, 2-Methylpropylcarbonylamino und 1,1-Dimethylethylcarbonylamino, insbesondere Methylcarbonylamino;
N-C₁-C₄-Alkylcarbonyl-N-methyl-amino wie N-Methylcarbonyl-N-methyl-amino, N-Ethylcarbonyl-N-methyl-amino, N-n-Propylcarbonyl-N-methyl-amino, N-1-Methylethylcarbonyl-N-methyl-amino, N-n-Butylcarbonyl-N-methyl-amino, N-1-Methylpropylcarbonyl-N-methyl-amino, N-2-Methylpropylcarbonyl-N-methyl-amino und N-1,1-Dimethylethylcarbonyl-N-methyl-amino, insbesondere N-Methylcarbonyl-N-methylamino.

Z steht beispielsweise für:
5- oder 6-gliedriger heterocyclischer, gesättigter oder ungesättigter Rest, enthaltend ein bis drei Heteroatome, ausgewählt aus der Gruppe Sauerstoff, Schwefel oder Stickstoff, beispielsweise fünfring Heteroaromaten wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, 1,2,4-Thiadiazol-3-yl, 1,2,4-Thiadiazol-5-yl, 1,3,4-Thiadiazol-2-yl, 1,2,3-Thiadiazol-4-yl, 1,2,3-Thiadiazol-5-yl, 1,2,5-Thiadiazol-3-yl, 1,2,4-Triazol-3-yl, , 1,3,4-Triazol-2-yl,1,2,3-Triazol-4-yl, 1,2,3-Triazol-5-yl, 1,2,4-Triazol-5-yl, Tetrazol-5-yl, insbesondere 2-Thiazolyl und 3-Isoxazolyl;
sechsring Heteroaromaten wie 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-5-yl und 1,2,4-Triazin-3-yl, 1,2,4-Triazin-6-yl, 1,2,4,5-Tetrazin-3-yl;
5- bis 6-gliedrige, gesättigte oder teilweise ungesättigte Heterocyclen, enthaltend ein bis drei Stickstoffatome und/oder ein oder zwei Sauerstoff- oder Schwefelatom wie 2-Tetrahydrofuranyl, 3-Tetrahydrofuranyl, 2-Tetrahydrothienyl, 3-Tetrahydrothienyl, Tetrahydrothiopyran-2-yl, Tetrahydrothiopyran-3-yl, Tetrahydrothiopyran-4-yl, 1,3-Dithiolan-2-yl, 1,3-Dithiolan-4-yl, 1-3-Dithian-2-yl, 1,3-Dithian-4-yl, 5,6-Dihydro-4H-1,3-thiazin-2-yl, 1,3-Oxathiolan-2-yl, 1,3-Oxathian-2-yl, 1-Pyrrolidinyl, 2-Pyrrolidinyl, 3-Pyrrolidinyl, 3-Isoxazolidinyl, 4-Isoxazolidinyl, 5Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl,1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl,2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl,2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 1,3-Dioxolan-2-yl, 1,3-Dioxolan-4-yl, 1,3-Dioxan-5-yl, 1,4-Dioxan-2-yl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl, insbesondere 2-Tetrahydrofuranyl, 1,3-Dioxolan-2-yl und 1,3-Dioxan-2-yl,
der gegebenenfalls durch
Halogen wie vorstehend genannt, insbesondere Fluor oder Chlor,
Cyano, Nitro,
eine Gruppe -COR⁸, beispielsweise Alkylcarbonyl wie vorstehend genannt, Alkoxycarbonyl wie vorstehend genannt, N-Alkylcarbamoyl wie vorstehend genannt, Dialkylcarbamoyl wie vorstehend genannt;
C₁-C₄-Alkyl wie vorstehend genannt,
C₁-C₄-Halogenalkyl wie beispielsweise Chlormethyl, Difluormethyl, Dichlormethyl, Trifluormethyl, Trichlormethyl, Chlordifluormethyl, 1-Fluorethyl, 2-Fluorethyl, 2,2-Difluorethyl, 1,1,2,2-Tetrafluorethyl, 2,2,2-Trifluorethyl, 2-Chlor-1,1,2-trifluorethyl und Pentafluorethyl, Decafluorbutyl, 1,1-Bis-trifluormethyl-2,2,2-trifluorethyl, bevorzugt Difluormethyl, Trifluormethyl, Trichlormethyl und Chlordifluormethyl;
C₃-C₈-Cycloalkyl, wie beispielsweise Cyclopropyl, Cyclobutyl, Cyclopentyl, Cyclohexyl, Cycloheptyl, Cyclooctyl, insbesondere Cyclopropyl und Cyclohexyl;
C₁-C₄-Alkoxy wie vorstehend genannt,
C₁-C₄-Halogenalkoxy wie beispielsweise Chlormethoxy, Dichlormethoxy, Trichlormethoxy, Fluormethoxy, Difluormethoxy, Trifluormethoxy, Chlordifluormethoxy, Dichlorfluormethoxy, 1-Fluormethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere C₁-C₃-Halogenalkoxy wie 2,2,2-Trifluorethoxy und 2-Chlor-2,2-difluorethoxy;
C₁-C₄-Alkylthio wie vorstehend genannt,
C₁-C₄-Halogenalkylthio wie vorstehend genannt,
Di-C₁-C₄-Alkylamino wie beispielsweise Dimethylamino, Diethylamino, Dipropylamino, Dibutylamino, N-Methyl-N-ethylamino, N-Methyl-N-propylamino, N-Methyl-N-1-methylethylamino, N-Methyl-N-1,1-Dimethylethylamino, Di-1-Methylethylamino, N-Ethyl-N-1-methylethylamino und N-Ethyl-N-1,1-dimethylethylamino;
gegebenenfalls substituiertes Phenyl
oder eine Oxogruppe, die gegebenenfalls auch in der tautomeren Form als Hydroxygruppe vorliegen kann, substituiert ist, beispielsweise Thiazolin-4,5-dion-2-yl, 3-Oxo-3H-1,2,4-dithiazolyl oder 2-Oxo-2H-1,3,4-dithiazolyl.
Benzokondensierte 5- oder 6-Ring-Heteroaromaten sind beispielsweise Benzofuranyl, Benzothienyl, Indolyl, Benzoxazolyl, Benzisoxazolyl, Benzthiazolyl, Benzisothiazolyl, Benzpyrazolyl, Indazolyl, 1,2,3-Benzothiadiazolyl, 2,1,3-Benzothiadiazolyl, Benzotriazolyl, Benzofuroxanyl, Chinolinyl, Isochinolinyl, Cinnolinyl, Chinazolinyl, Chinoxalinyl oder Phthalazinyl. Beispiele für besonders bevorzugte Verbindungen der allgemeinen Formel I sind in den folgenden Tabellen 1 bis 5 zusammengestellt.

Beispiele für besonders bevorzugte Verbindungen der allgemeinen Formel I sind in der folgenden Tabelle zusammengestellt.

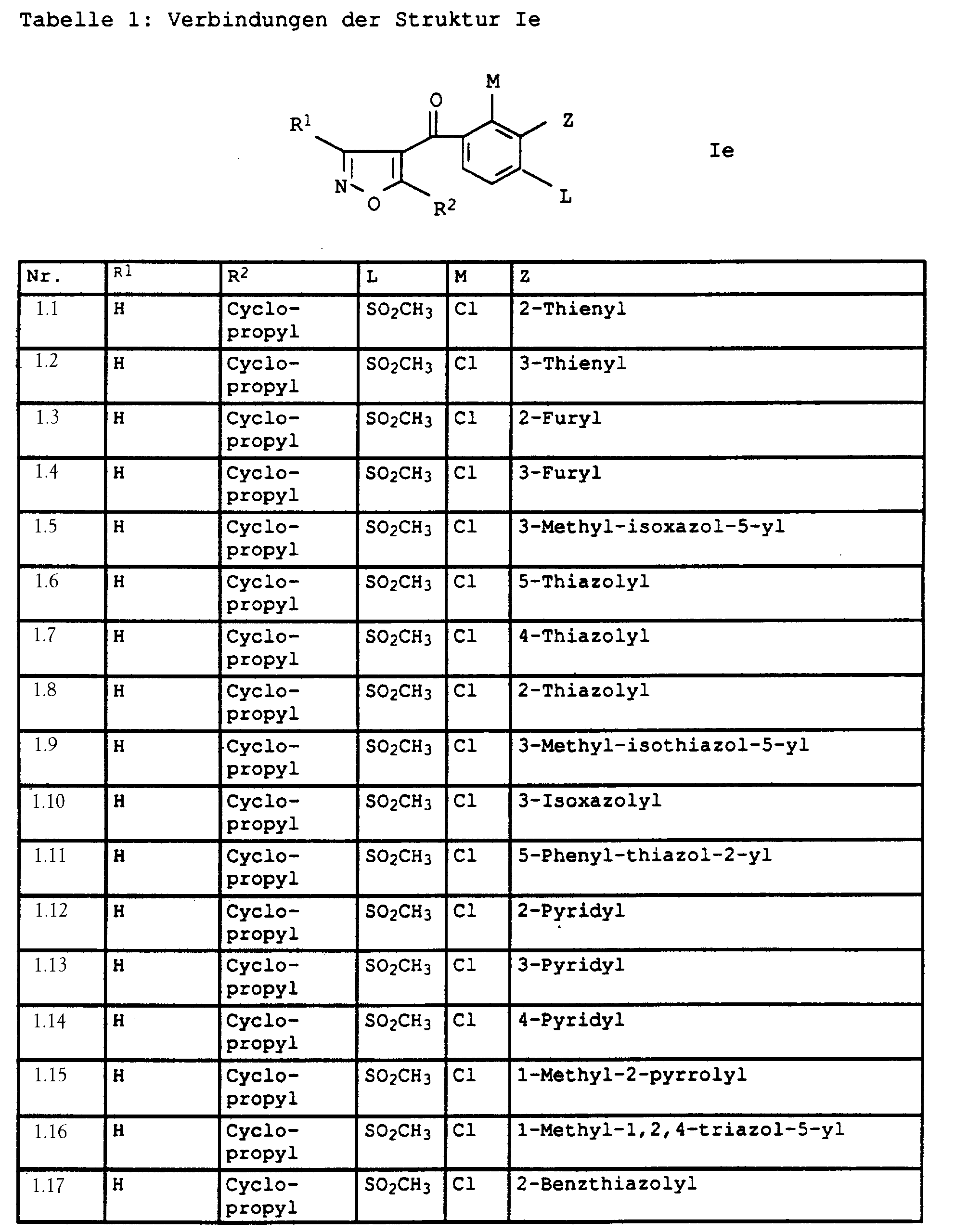

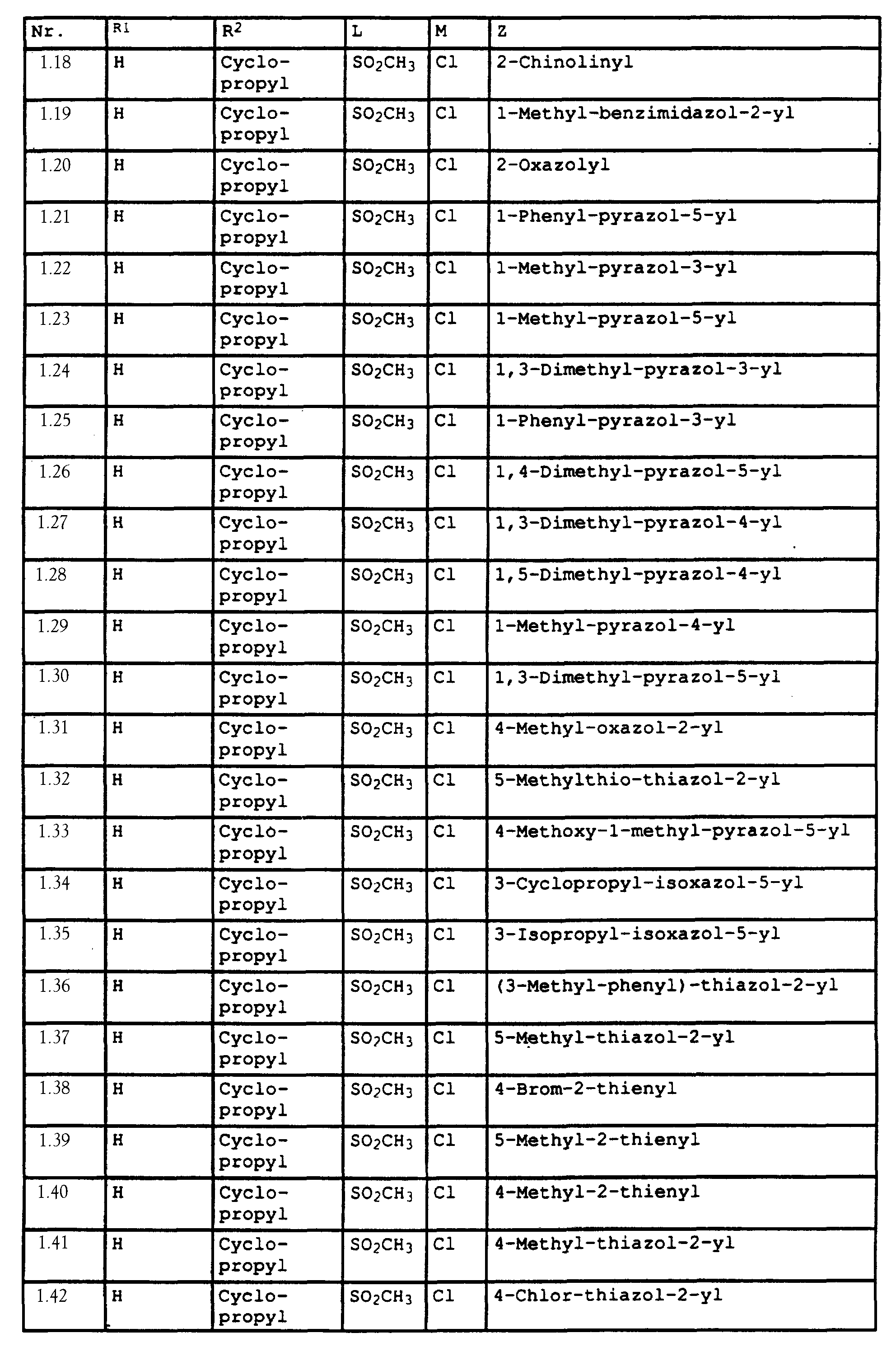

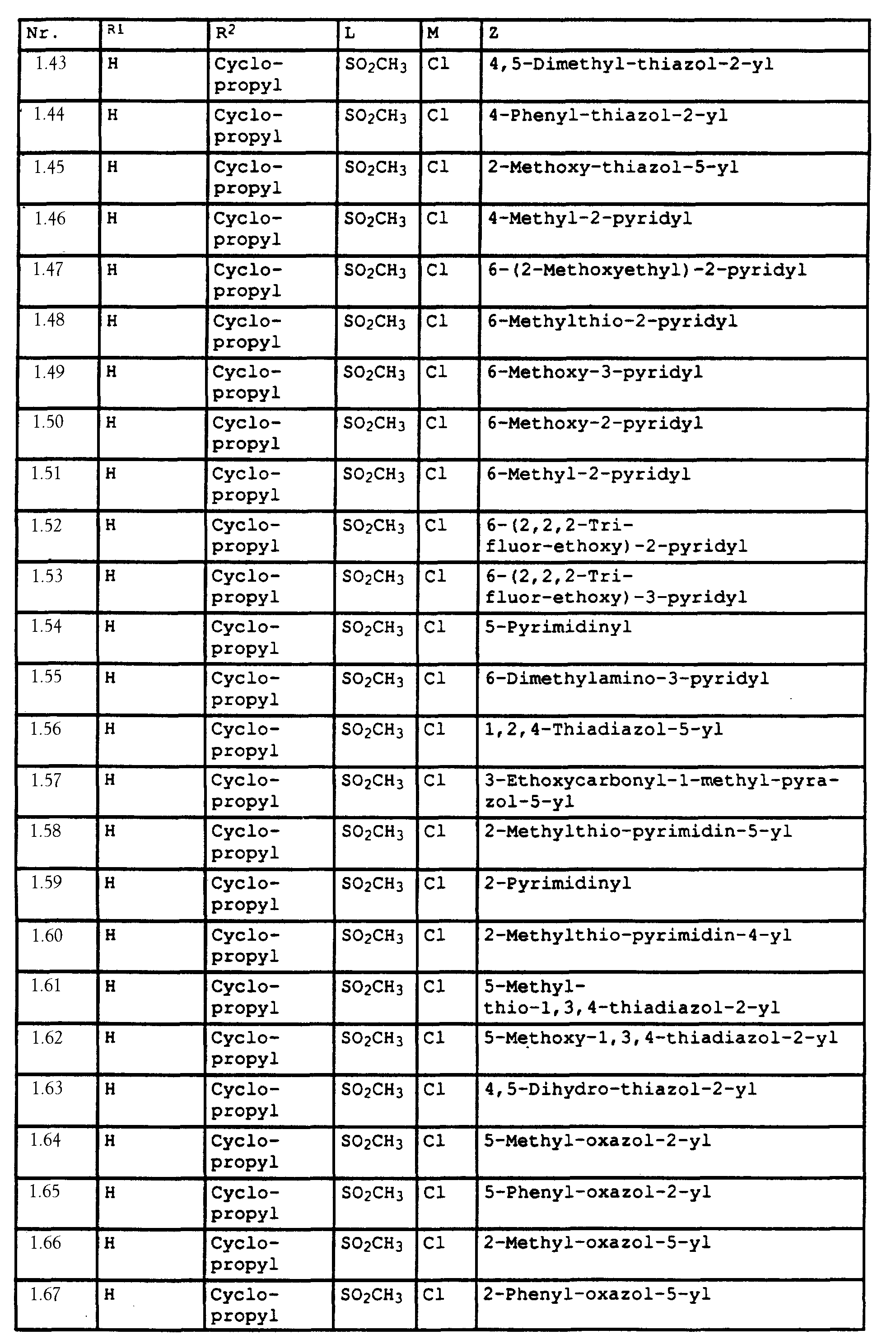

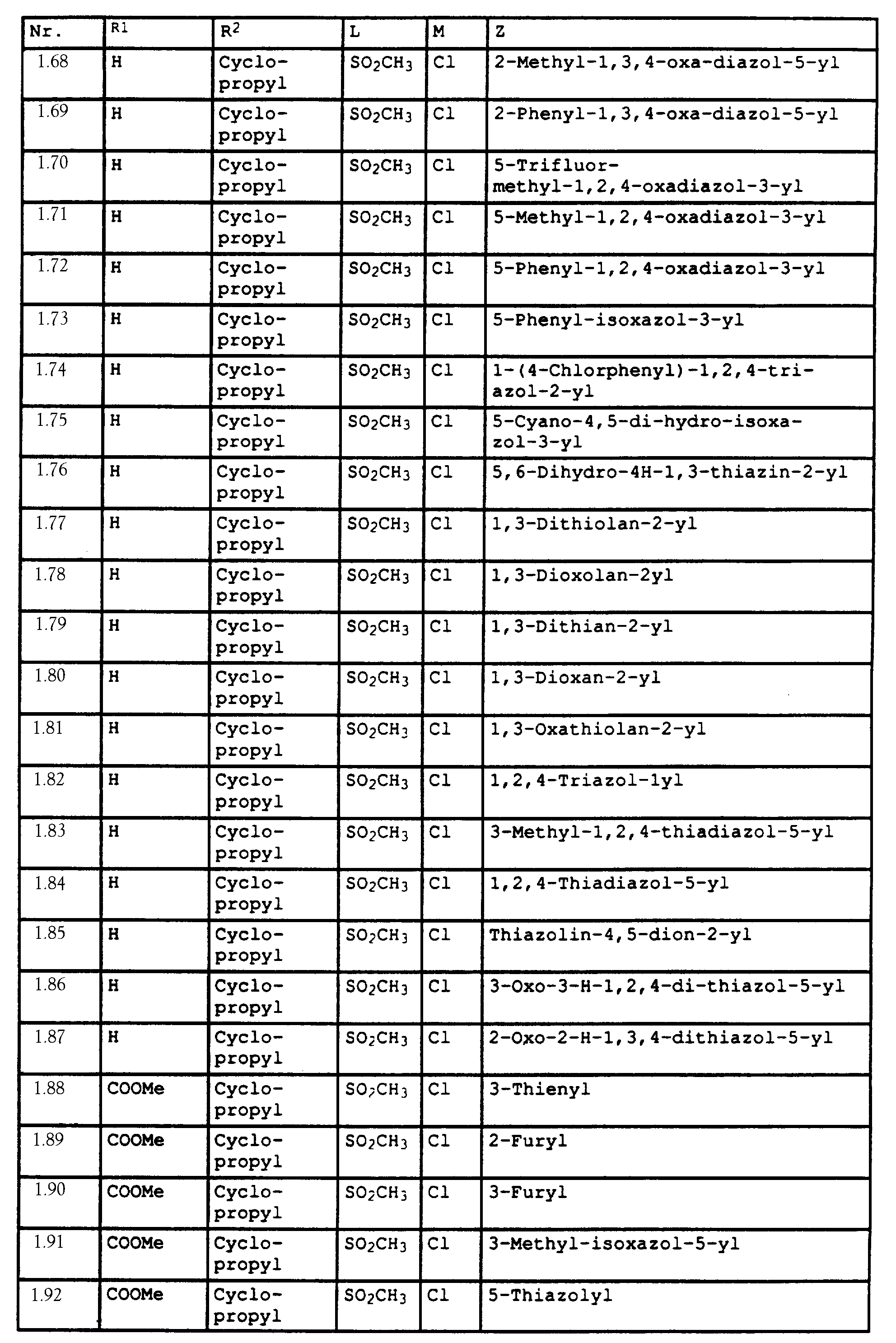

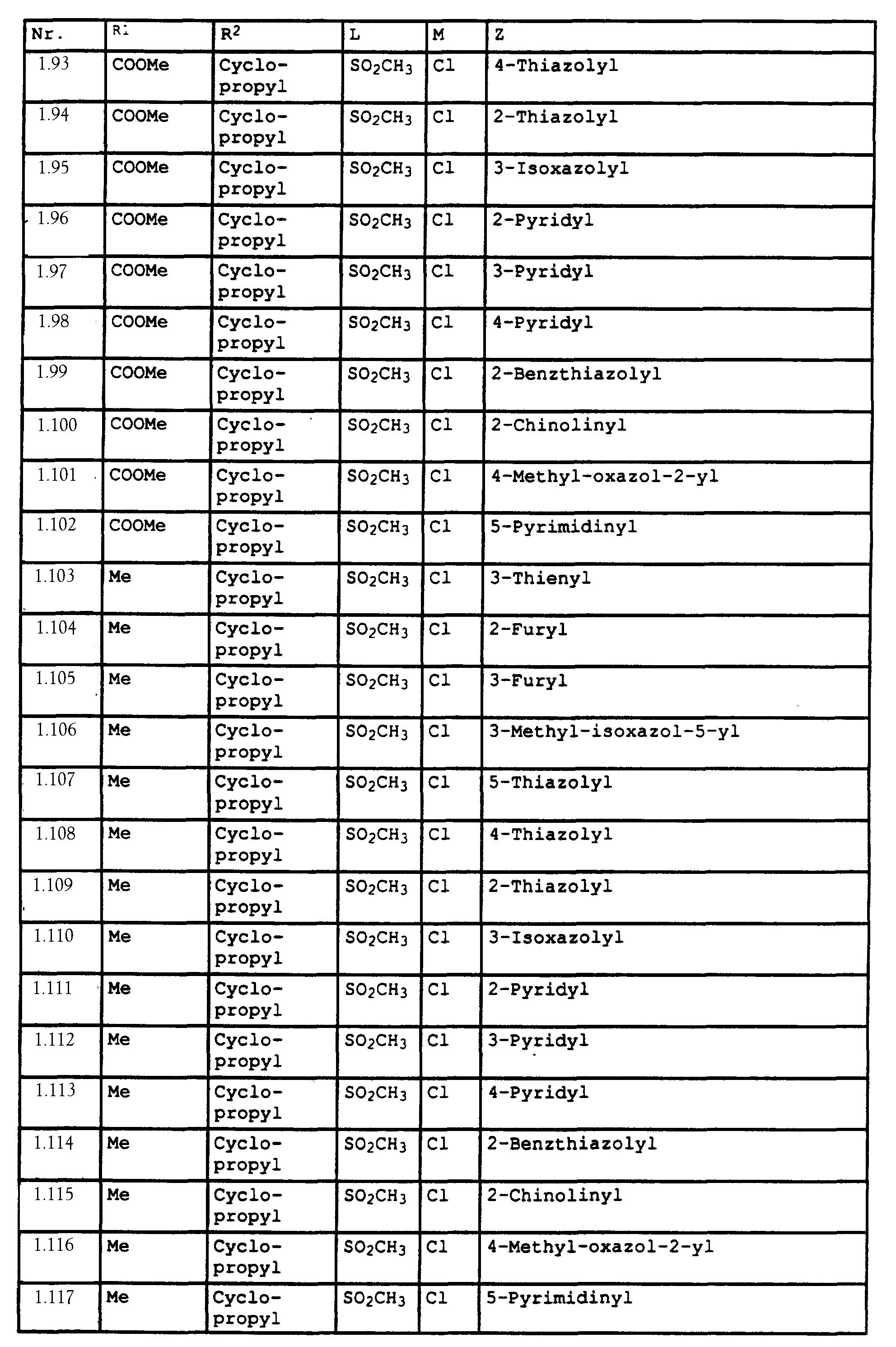

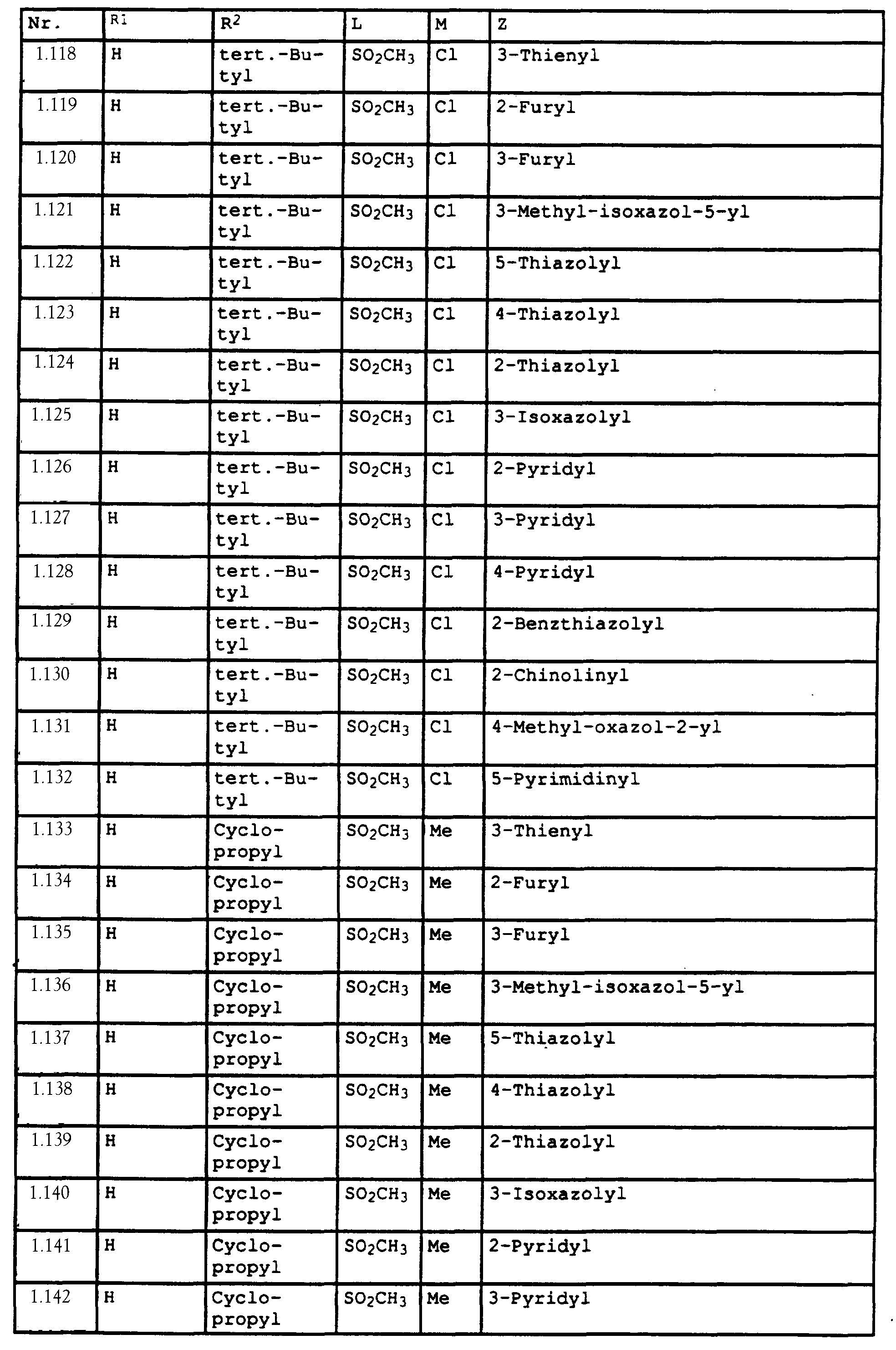

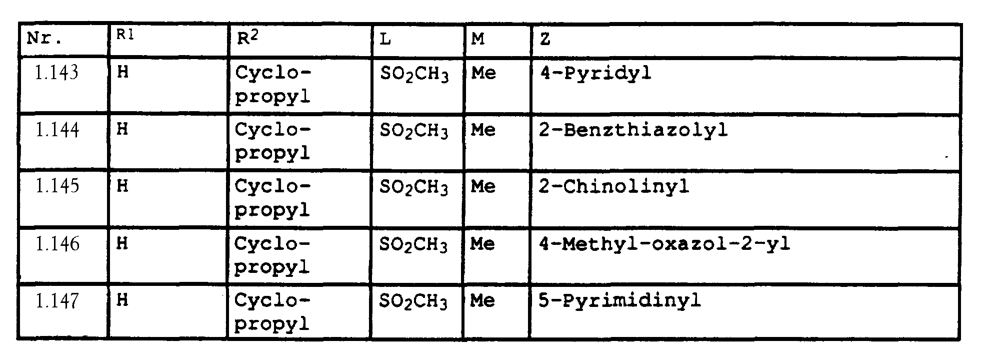

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus off icinalis, Beta vulgaris spp. altissima, Beta vulgaris SPP. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossyplum hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp , Manihot esculenta, Medicago sativa, Musa spp., Nicotiana tabacum (N. rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen 1 auch in Kulturen, die durch Züchtung einschließlich gentechnischer Methoden gegen die Wirkung von Herbiziden tolerant sind, verwandt werden. Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin und Dibutylphthalinsulfonsäure, sowie von Fettsäuren, Alkyl- und Alkylarylsulfonaten, Alkyl-, Lauryl- ether- und Fettalkoholsulfaten- sowie Salze sulfatierter Hexa-, Hepta und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.%, Wirkstoff. Die Wirkstoffe werden dabei in einer Reinheit von 90 % bis 100 %, vorzugsweise 95 % bis 100 % (nach NMR-Sektrum) eingesetzt.

Die erfindungsgemäßen Verbindungen I können beispielsweise wie folgt formuliert werden:
- I: 20 Gewichtsteile der Verbindung Nr. 1.8 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II: 20 Gewichtsteile der Verbindung Nr. 1.8 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctyphenyl und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- III: 20 Gewichtsteile des Wirkstoffs Nr. 1.8 werden in einer Mischung gelöst, die aus 25 Gewichtsteilen Cyclohexanon, 65 Gewichtsteilen einer Mineralölfraktion vom Siedepunkt 210 bis 280°^{C} und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- IV: 20 Gewichtsteile des Wirkstoffs Nr. 1.8 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew. % des Wirkstoffs enthält.
- V: 3 Gewichtsteile des Wirkstoffs Nr. 1.8 werden mit 97 Gewichtsteilen feinteiligem Kaolin vermischt. Man erhält auf diese Weise ein Stäubemittel, das 3 Gew. % des Wirkstoffs enthält.
- VI: 20 Gewichtsteile des Wirkstoffs Nr. 1.8 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
- VII: 1 Gewichtsteil der Verbindung Nr. 1.8 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII: 1 Gewichtsteil der Verbindung Nr. 1.8 wird in einer Mischung gelöst, die aus 80 Gewichtsteilen Cyclohexanon und 20 Gewichtsteilen Emulphor EL besteht. Man erhält ein stabiles Emulsionskonzentrat.

Zur Verbreiterung des Wirkungsspektrums und zur Erzielung synergistischer Effekte können die lsoxazolyl-Benzoylderivate I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäure derivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a.S.).

### Anwendungsbeispiele

Die herbizide Wirkung der Isoxazolyl-Benzoylderivate der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung werden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10 - 25 C bzw. 20 - 35 C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

### Herstellungsbeispiele

### A) Herstellung der Ausgangsstoffe

1. 2-Chlor-3-formyl-4-methylsulfonyl-benzoesäuremethylester
   a. Zu einer Suspension von 286 g (2.14 mol) Aluminiumtrichlorid in 420 ml 1,2-Dichlorethan wurde bei 15-20°C eine Lösung von 157 g (2 mol) Acetylchlorid in 420 mol 1,2-Dichlorethan getropft. Anschließend wurde eine Lösung von 346 g (2 mol) 2-Chlor-6-methylthio-toluol in 1 l 1,2-Dichlorethan zugetropft. Nach 12 Stunden Nachrühren wurde das Reaktionsgemisch in eine Mischung aus 3 l Eis und 1 l konz. HCl gegossen. Es wurde mit Methylenchlorid extrahiert, die organische Phase mit Wasser gewaschen, mit Natriumsulfat getrocknet und eingeengt. Der Rückstand wurde im Vakuum destilliert.
      Man erhielt 256 g (60 % d.Th.) 2-Chlor-3-methyl-4-methylthio-acetophenon,
      Fp.: 46°C
   b. 163 g (0.76 mol) 2-Chlor-3-methyl-4-methylthio-acetophenon wurden in 1,5l Eisessig gelöst , mit 18,6 g Natriumwolframat versetzt und unter Kühlung 173,3 g 30 %ige Wasserstoffperoxidlösung zugetropft. Es wurde 2 Tage nachgerührt und anschließend mit Wasser verdünnt. Der ausgefallene Feststoff wurde abgesaugt, mit Wasser gewaschen und getrocknet.
      Man erhielt 164 g (88% d. Th.) 2-Chlor-3-methyl-4-methylsulfonyl-acetophenon, Fp.: 110-111°C
   c. 82 g (0.33 mol) 2-Chlor-3-methyl-4-methylsulfonyl-acetophenon wurden in 700 ml Dioxan gelöst und bei Raumtemperatur mit 1 l einer 12,5 %igen Natriumhypochloritlösung versetzt. Anschließend wurde 1 Stunde bei 80°C nachgerührt. Nach dem Abkühlen bildeten sich zwei Phasen, von denen die untere mit Wasser verdünnt und schwach angesäuert wurde. Der ausgefallene Feststoff wurde mit Wasser nachgewaschen und getrocknet.
      Man erhielt 60 g (73 % d.Th) 2-Chlor-3-methyl-4-methylsulfonyl-benzoesäure, Fp.: 230-231°C.
   d. 100 g (0.4 mol) 2-Chlor-3-methyl-4-methylsulfonyl-benzoesäure wurden in 1 l Methanol gelöst und bei Rückflußtemperatur 5 Stunden mit HCl begast. Anschließend wird eingeengt.
      Man erhielt 88.5 g (84 % d.Th.) 2-Chlor-3-methyl-4-methylsulfonyl-benzoesäuremethylester, Fp.: 107-108°C
   e. 82 g (0.31 mol) 2-Chlor-3-methyl-4-methylsulfonyl-benzoesäuremethylester werden in 2l Tetrachlormethan gelöst und unter Belichtung portionsweise mit 56 g (0.31 mol) N-Bromsuccinimid versetzt. Das Reaktionsgemisch wurde filtriert, das Filtrat eingeengt und der Rückstand in 200 ml Methyl-tert.-butylether aufgenommen. Die Lösung wird mit Petrolether versetzt, der ausgefallene Feststoff abgesaugt und getrocknet.
      Man erhielt 74,5 g (70 % d.Th) 3-Brommethyl-2-chlor-4-methylsulfonyl-benzoesäuremethylester, Fp.: 74-75°C.
   f. Eine Lösung von 41 g (0.12 mol) 3-Brommethyl-2-chlor-4-methylsulfonyl-benzoesäuremethylester in 250 ml Acetonitril wurde mit 42,1 g (0.36 mol) N-Methylmorpholin-N-oxid versetzt. Der Ansatz wurde 12 Stunden bei Raumtemperatur nachgerührt, anschließend eingeengt und der Rückstand in Essigester aufgenommen. Die Lösung wurde mit Wasser extrahiert, mit Natriumsulfat getrocknet und eingeengt.
      Man erhielt 31,2 g (94 % d.Th.) 2-Chlor-3-formyl-4-methylsulfonyl-benzoesäuremethylester, Fp.: 98-105°C
2. 2-Chlor-4-methylsulfonyl-3-(trifluormethylsulfonyl)oxy-benzoesäure-methylester
   a. 101 g (0.41 mol) 2-Chlor-3-hydroxy-4-methylsulfonyl-benzoesäure werden in 1,3l Methanol gelöst und unter Rückfluß 4 Stunden mit HCl begast. Die Lösung wurde eingeengt, der Rückstand mit Dichlormethan aufgenommen und mit K₂CO₃-Lösung extrahiert. Die wäßrige Phase wurde mit verdünnter Salzsäure auf pH 7 eingestellt und mit Dichlormethan gewaschen. Anschließend wurde auf pH 1 angesäuert und das Produkt mit Dichlormethan extrahiert.
      Man erhielt 76,2 g (71 % d.Th.) 2-Chlor-3-hydroxy-4-methylsulfonyl-benzoesäuremethylester.
   b. Eine Lösung aus 76 g (0,29 mol) 2-Chlor-3-hydroxy-4-methylsulfonyl-benzoesäuremethylester und 68 g Pyridin in 700 ml Dichlormethan wurde bei -20°C mit 89 g (0.32 mol) Trifluormethansulfonsäureanhydrid versetzt. Die Lösung wurde 12 Stunden bei Raumtemperatur nachgerührt, mit Dichlormethan verdünnt und mit Wasser extrahiert. Die organische Phase wurde über Magnesiumsulphat getrocknet und eingeengt.
      Man erhielt 94 g (82 % d.Th) 2-Chlor-4-methylsulfonyl-3-(trifluormethylsulfonyl)oxy-benzoesäure-methylester, Fp.: 69°C.

### B) Herstellung der Zwischenprodukte

1. 3-(3-Isopropylisoxazol-5-yl)-4-methylsulfonyl-benzoesäuremethylester
   a. 30 g (102 mmol) 3-Brom-4-methylsulfonyl-benzoesäuremethylester, 90 mg Palladiumdichlorid und 240 mg Triphenylphosphin in 200 ml Diethylamin und 60 ml Dimethylformamid werden mit 10 g (102 mmol) (Trimethylsilyl)-acetylen und 180 mg Kupfer-I-jodid versetzt und 4,5 Stunden bei 40°C gerührt. Anschließend wurde noch 12 Stunden bei Raumtemperatur nachgerührt. Das Reaktionsgemisch wurde filtriert, das Filtrat eingeengt und der Rückstand über Kieselgel mit Toluol als Laufmittel chromatographiert.
      Man erhielt 17,3g (55% d.Th.) 4-Methylsulfonyl-3-(trimethylsilyl)ethinyl-benzoesäuremethylester als Öl.
   b. 25 g 4-Methylsulfonyl-3-(trimethylsilyl)ethinyl-benzoesäuremethylester werden mit 100 ml Methanol und 0,9 g Kaliumkarbonat 18 Stunden bei Raumtemperatur gerührt. Anschließend wurde vom Feststoff abgesaugt, eingeengt und mit Essigester/Wasser extrahiert. Die organische Phase wurde über Natriumsulfat getrocknet und eingeengt.
      Man erhielt 15 g (79 % d.Th.) 4-Methylsulfonyl-3-ethinylbenzoesäure-methylester, Fp.: 95-98°C.
   c. 13,5 g (57 mmol) 4-Methylsulfonyl-3-ethinyl-benzoesäuremethylester werden in 50 ml Dichlormethan gelöst, mit 5,2g (60 mmol) Isobutyraldehydoxim versetzt und 41 g einer 12,5 %igen Natriumhypochloritlösung zugetropft. Anschließend wurde 24 Stunden bei Raumtemperatur nachgerührt. Der Reaktionsansatz wurde anschließend mit Dichlormethan/Wasser extrahiert, die organische Phase eingeengt und der Rückstand über Kieselgel mit Toluol/ Essigester als Laufmittel chromatographiert.
      Man erhielt 8,8 g (48 % d.Th) 3-(3-Isopropylisoxazol-5-yl)-4-methylsulfonyl-benzoesäuremethylester, Fp.: 102-104°C.
2. 2-Chlor-3-(isoxazol-3-yl)-4-methylsulfonyl-benzoesäuremethylester
   a. 15 g ( 54 mmol) 2-Chlor-3-formyl-4-methylsulfonyl-benzoesäuremethylester (Beisp. A.1.) und 4,2 g ( 60 mmol) Hydroxylaminhydrochlorid werden mit 300 ml Methanol gerührt und eine Lösung von 3,18 g (30 mmol) Natriumcarbonat in 80 ml Wasser zugetropft. Die Reaktionsmischung wird über Nacht bei Raumtemperatur gerührt, anschließend wird das Methanol abdestilliert und der Ansatz mit Ether/Wasser extrahiert. Die Etherphase wird mit Natriumsulfat getrocknet und eingeengt.
      Man erhält 14,4 g (91% d.Th.) 2-Chlor-3-hydroxyiminomethyl-4-methylsulfonyl-benzoesäuremethylester,
      Fp.: 126-128 °C.
   b. 5,3 g ( 18 mmol) 2-Chlor-3-hydroxyiminomethyl-4-methylsulfonyl-benzoesäuremethylester werden in 50 ml Dichlormethan gelöst und bei 0-5°C 30 Minuten lang Acetylen eingeleitet. Anschließend wird mit mit einer Spatelspitze Natriumacetat versetzt und 15ml einer 10%igen Natriumhypochlorit-Lösung bei 10°C unter weiterer Acetylen-Einleitung zutropft. Nach beendeter Zugabe wird für weitere 15 Minuten Acetylen bei 10°C eingeleitet und anschließend 12 Stunden nachgerührt. Danach werden die Phasen getrennt, die organische Phase mit Wasser gewaschen, mit Natriumsulfat getrocknet und eingeengt.
      Man erhält 4,8 g (84 % d.Th.)
      2-Chlor-3-(isoxazol-3-yl)-4-methylsulfonyl-benzoesäuremethylester, Fp.: 145-147°C.
3. 2-Chlor-3-(thiazol-2-yl)-4-methylsulfonyl-benzoesäuremethylester
   33 g (88 mmol) 2-(Tributylstannyl)-thiazol, 17,5 g (44 mmol) 2-Chlor-4-methylsulfonyl-3-(trifluormethylsulfonyl)oxy-benzoesäure-methylester (Beisp. A.2.) , 5,8 g Lithiumchlorid, 1g Tetrakis-(triphenylphosphin)-palladium-(0), eine Spatelspitze 2,6-Di-tert.-butyl-4-methyl-phenol und 200 ml 1,4-Dioxan werden in einem Autoklaven 3 Stunden bei 140 °C unter Eigendruck gerührt. Nach dem Abkühlen wird die Reaktionsmischung über eine Kieselgelschicht abfiltriert, mit Methyl-tert.-butylether nachgewaschen und eingeengt. Der Rückstand wird über Kieselgel mit Toluol/Essigester als Laufmittel chromatographiert.
   Man erhält 9,1 g (62,6% d.Th.) 2-Chlor-3-(thiazol-2-yl)-4-methylsulfonyl-benzoesäuremethylester, Fp.: 135-138 °C.
4. 2-Chlor-3-(oxazol-5-yl)-4-methylsulfonyl-benzoesäuremethylester
   25 g (0,09 mol) 2-Chlor-3-formyl-4-methylsuflonyl-benzoesäuremethylester (Beisp. A.1.), 17,6 g (0,09 mol) Tosylmethylenisocyanid und 6,2 g (0,045 mol) fein gepulvertes Kaliumcarbonat werden mit 450 ml Methanol 5 Stunden bei Rückflußtemperatur gerührt. Anschließend wird das Lösungsmittel abgezogen, der Rückstand in Essigester aufgenommen und mit Wasser extrahiert. Die Essigesterphase wird mit Natriumsulfat getrocknet und eingeengt.
      Man erhält 24,7 g (87 % d.Th.) 2-Chlor-3-(oxazol-5-yl)-4-methylsulfonylbenzoesäuremethylester, ¹H-NMR (CDCl₃)
      δ: 8,24 (d,1H), 8,15 (s,1H), 8,01 (d,1H), 7,40 (s,1H), 4,0 (s,3H), 2,96 (s,3H)

In analoger Weise werden die in der nachfolgenden Tabelle aufgeführten Zwischenprodukte erhalten:

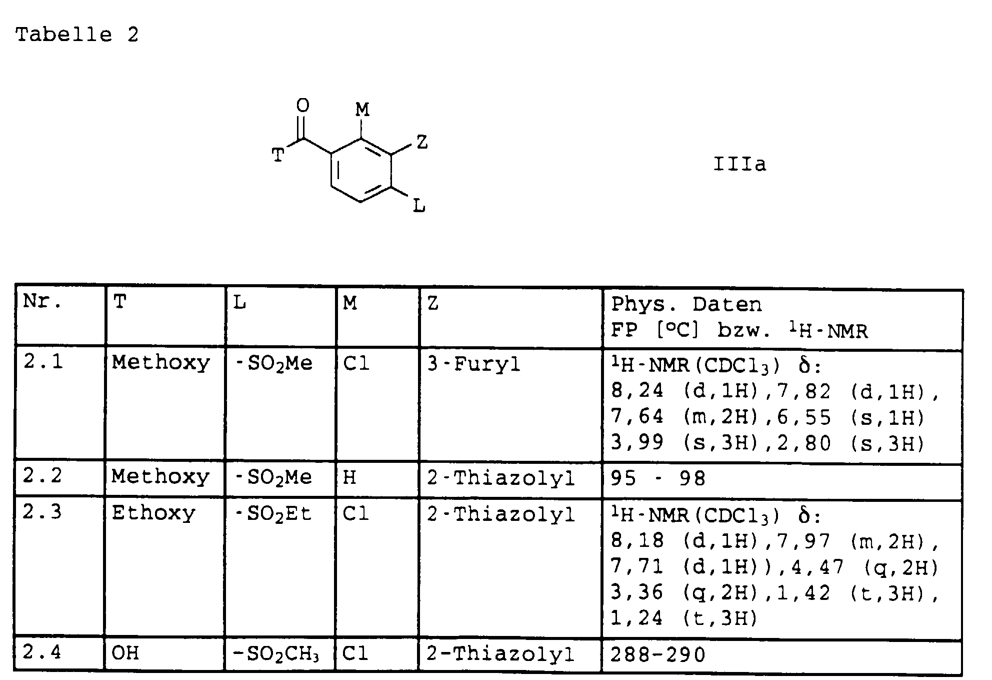

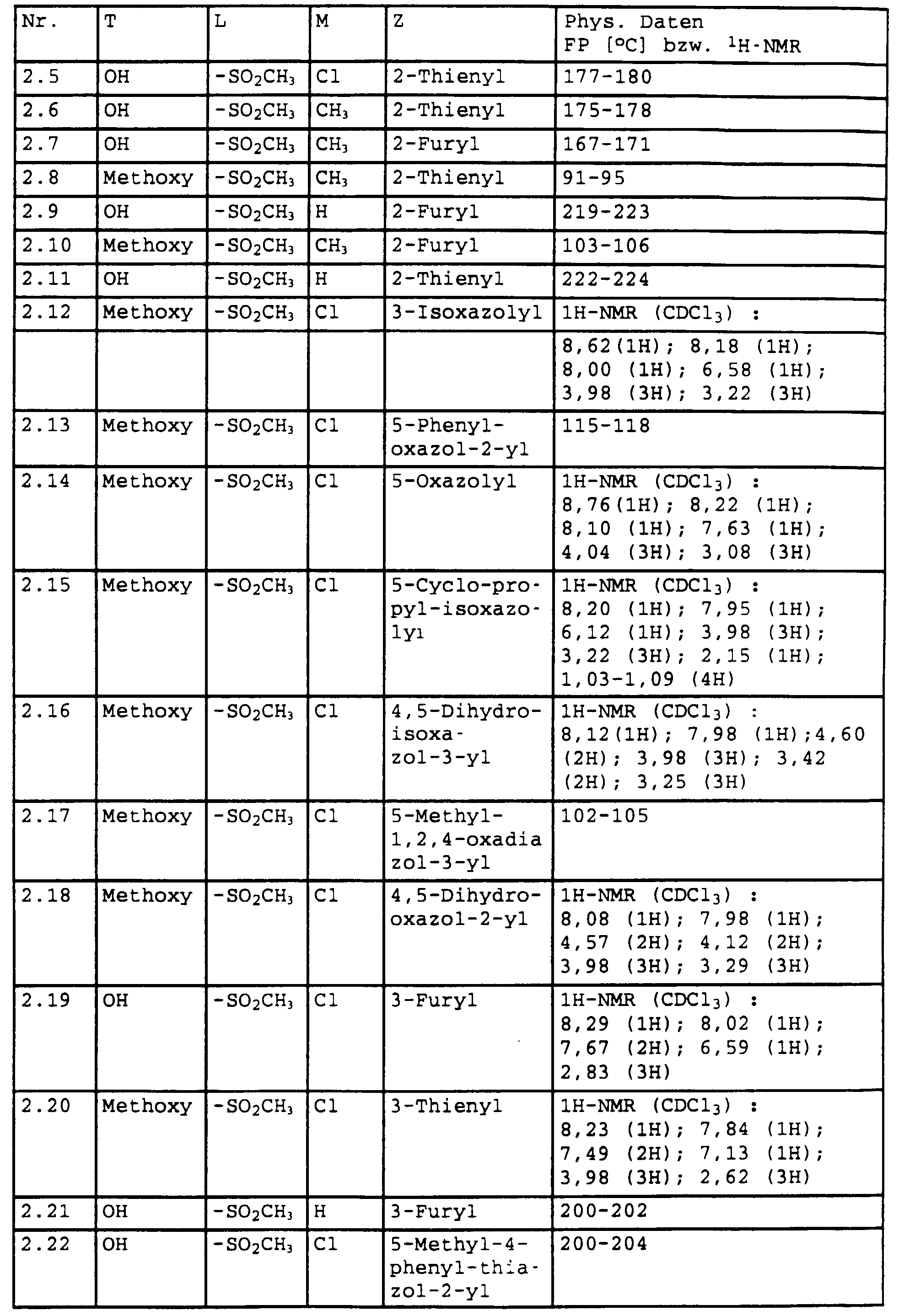

### C) Herstellung der Endprodukte

1.
   a. 8 g (25 mmol) 2-Chlor-4-methylsulfonyl-(3-thiazol-2-yl)benzoesäuremethylester werden in 50 ml Methanol gelöst und mit 1,5 g (37 mmol) NaOH versetzt. Die Lösung wird 12 Stunden bei Raumtemperatur gerührt. Anschließend wird das Reaktionsgemisch eingeengt, der Rückstand in Wasser aufgenommen u4nd mit Salzsäure angesäuert. Nach längerem Rühren bilden sich hellgelbe Kristalle. Der Feststoff wird abgesaugt und getrocknet.
      Man erhält 6,6 g (86% d.Th.) 2-Chlor-4-methylsulfonyl-(3-thiazol-2-yl)-benzoesäure, Fp.: 288-290 °C.
   b. 6 g (19 mmol) 2-Chlor-4-methylsulfonyl-(3-thiazol2-yl)-benzoesäure werden in 60 ml Toluol gelöst, mit einem Tropfen Dimethylformamid versetzt und 3,2 g (27 mmol) Thionylchlorid zugegeben. Nach 4 Stunden Refluxieren wird das Reaktionsgemisch eingeengt.
      Man erhält 6,3 g (99 % d.Th.) 2-Chlor-4-methylsulfonyl-(3-thiazol-2-yl)-benzoesäurechlorid, Fp.:
      97-98°C.
   c. Zu einer Suspension von 2,55 g (0,023 Mol) Calciumchlorid (gepulvert) in 150 ml Aceton werden nacheinander 4,2g (0,023 Mol) 3-Oxo-3-cyclopropyl-propionsäure-tert.butylester und 4,65g (0,046 Mol) Triethylamin zugegeben. Nach einer Stunde rühren bei Raumtemperatur werden 7,65g (0,023 Mol) 2-Chlor-4-methylsulfonyl-(3-thiazol-2-yl)benzoesäurechlorid in 75 ml Aceton zugetropft. Es wird 3 Std. bei 45 °C gerührt und anschließend das Lösungsmittel i.Vak. entfernt. Der Rückstand wird in Essigester gelöst und mit 1n Salzsäure behandelt. Die Essigesterphase wird über Kieselgel filtriert und eingeengt. Der kristalline Rückstand wird in 200 ml Toluol gelöst, mit 0,7g p-Toluolsulfonsäure versetzt und 3 Std am Rückfluß gekocht. Es wird abgekühlt, über Kieselgel filtriert und eingeengt.
      Man erhält 5,85g (67 % d.Th) 1-[2-Chlor-4-methylsulfonyl-(3-thiazol-2-yl)-phenyl] - 3-cyclopropyl-propan-1,3-dion, Fp.: 146-147°C.
   d. Eine Mischung von 2,4g (0,006 Mol) 1-[2-Chlor-4-methylsulfonyl-(3-thiazol-2-yl)-phenyl]-3-cyclopropyl-propan-1,3-dion, 0.96g (0,009 Mol) Acetanhydrid und 20 ml Triethylorthoformiat werden 3 Std bei 110°C gerührt und anschließend eingeengt. Das Produkt wird säulenchromatographisch mit Toluol/Essigester als Eluent gereinigt.
      Man erhält 2,4g (93 % d.Th.) 1-[2-Chlor-4-methylsulfonyl-(3-thiazol-2-yl)-phenyl]-3-cyclopropyl-2-hydroxymethylenpropan-1,3-dion, Fp.: 176°C.
   e. 2,4 g (0, 006 Mol) 1-[2-Chlor-4-methylsulfonyl(3-thiazol-2-yl)-phenyl]-3-cyclopropyl-2-hydroxymethylenpropan-1,3-dion werden mit 0,52g (0,007 Mol) Hydroxylamin Hydrochlorid in 60 ml Dichlormethan vorgelegt und bei Raumtemperatur eine Lösung von 4g Natriumcarbonat in 50 ml Wasser zugetropft. Die Mischung wird 15 Std bei Raumtemperatur gerührt. Anschließend werden die Phasen getrennt und die organische Phase eingeengt. Das Rohprodukt wird säulenchromatographisch mit Toluol/Essigester als Eluent gereinigt.
      Man erhält 1,2g (50 % d.Th.) 4-[2-Chlor-4-methylsulfonyl-(3-thiazol-2-yl)-benzoyl]-5-cyclopropylisoxazol, Fp.: 153-155 °C.

In analoger Weise werden die in der nachfolgenden Tabelle aufgeführten Verbindungen erhalten:

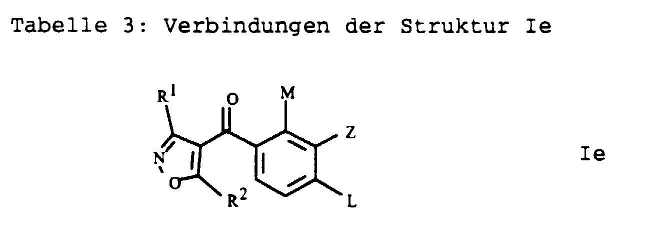

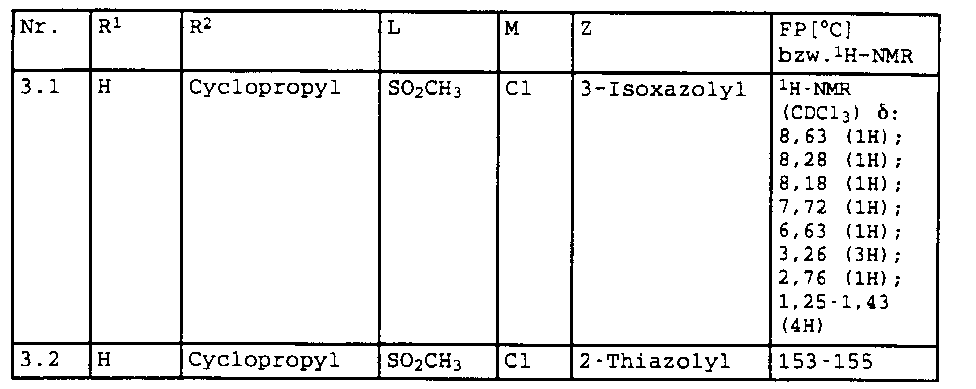

## Patentansprüche

1. Isoxazol-4-yl-benzoylderivate der Formel I 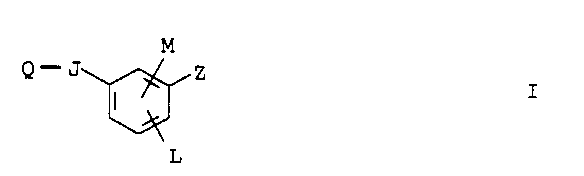 in der die Substituenten folgende Bedeutung haben:
L,M Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können, Halogen, Cyano, Nitro, eine Gruppe -(Y)ₙ-S(O)ₘR⁷ oder eine Gruppe -(Y)ₙ-CO-R⁸
Z ein 5- oder 6-gliedriger heterocyclischer, gesättigter oder ungesättigter Rest, enthaltend ein bis drei Heteroatome, ausgewählt aus der Gruppe Sauerstoff, Schwefel oder Stickstoff, der gegebenenfalls durch Halogen, Cyano, Nitro, eine Gruppe -CO-R⁸, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl oder eine Oxogruppe, die gegebenenfalls auch in der tautomeren Form als Hydroxygruppe vorliegen kann, substituiert ist oder der mit einem ankondensierten, gegebenenfalls durch Halogen, Cyano, Nitro, C₁-C₄-Alkyl oder C₁-C₄-Halogenalkyl substituierten Phenylring, einem ankondensierten Carbocyclus oder einem ankondensierten, gegebenenfalls durch Halogen, Cyano, Nitro, C₁-C₄-Alkyl, Di-C₁-C₄-Alkylamino, C₁-C₄-Alkoxy, C₁-C₄-Halogenalkoxy, oder C₁-C₄-Halogenalkyl substituierten zweiten Heterocyclus ein bicyclisches System bildet.
Y O, NR⁹
n null oder eins
m null, eins oder zwei
R⁷ C₁-C₄-Alkyl, C₁-C₄-Halogenalkyl oder NR⁹R¹⁰
R⁸ C₁-C₄-Alkyl, C₁-C₄-Halogenalkyl, C₁-C₄-Alkoxy, oder NR⁹R¹⁰
R⁹ Wasserstoff oder C₁-C₄-Alkyl
R¹⁰ C₁-C₄-Alkyl
Q ein in Stellung 4 verknüpfter Isoxazolring der Formel II 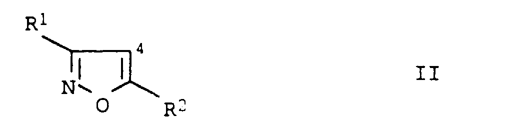
in welcher
R¹ für Wasserstoff oder -CO₂R³
R² für Wasserstoff, C₁-C₄-Alkyl oder C₃-C₆-Cycloalkyl, welches gegebenenfalls durch ein oder mehrere Halogenatome oder C₁-C₄-Alkyl substituiert sein kann und
R³ für C₁-C₄-Alkyl steht;
J ein Carbonylrest CO oder ein Rest -CHR⁴;
R⁴ eine OH-Gruppe, eine ggf. mit C₂-C₄-Acyl acylierte Hydroxylgruppe oder ein Chloratom bedeutet;
sowie landwirtschaftlich übliche Salze der Verbindungen I.

2. Isoxazol-4-yl-benzolderivate der Formel Ia gemäß Anspruch 1, 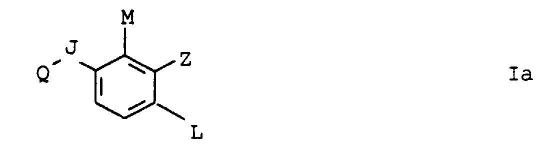 in der L für C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano und M für Wasserstoff, C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano steht und Q, J und Z die in Anspruch 1 angegebenen Bedeutungen haben.

3. Isoxazol-4-yl-benzoylderivate der Formel Ib gemäß Anspruch 1, 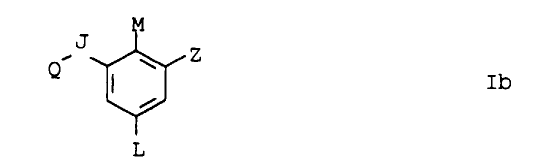 in der L und M für C₁-C₆-Alkyl, C₂-C₆-Alkenyl, C₂-C₆-Alkinyl, C₁-C₄-Alkoxy, C₁-C₄-Alkylthio, C₁-C₄-Halogenalkyl, C₁-C₄-Halogenalkoxy, C₁-C₄-Halogenalkylthio, C₁-C₄-Alkylsulfonyl, Halogen, Nitro oder Cyano stehen und Q, J und Z die in Anspruch 1 angegebenen Bedeutungen haben.

4. Isoxazol-4-yl-benzoylderivate der Formel I gemäß Anspruch 1 in der die Reste L bzw. M für Wasserstoff, Methyl, Methoxy, Methylthio, Chlor, Cyano, Methylsulfonyl, Nitro oder Trifluormethyl stehen.

5. Verfahren zur Herstellung der Isoxazol-4-yl-benzoylderivate der Formel I gemäß Anspruch 1, in der Q einen Rest CO-J darstellt, dadurch gekennzeichnet, daß man Alkinylstannate der Formel A2
(C₄H₉)Sn - C ≡ C- R² A2
mit einem Benzoesäurederivat der Formel III 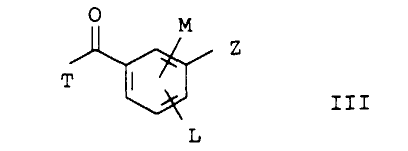 wobei T = Halogen bedeutet und L M und Z die in Anspruch 1 genannte Bedeutung haben, zu Benzoylalkinen der Formel B2 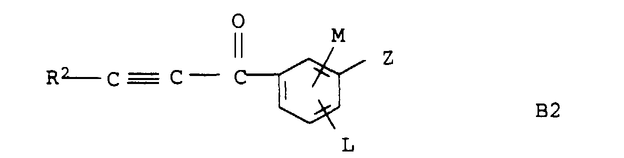 umsetzt und diese mit Nitriloxiden der Formel C2 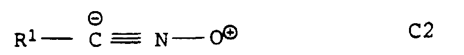 in der R¹ für C₁-C₄-Alkyl steht, einer Cycloaddition zu den Isoxazolen der Formel Ic 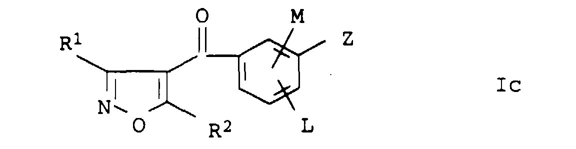 unterwirft.

6. Herbizides Mittel, enthaltend mindestens ein Benzoylderivat der Formel I gemäß Anspruch 1 und übliche inerte Zusatzstoffe.

7. Verfahren zur Bekämpfung unerwünschten Pflanzenwuchses, dadurch gekennzeichnet, daß man eine herbizid wirksame Menge eines Benzoylderivates der Formel I gemäß Anspruch 1 auf die Pflanzen oder deren Lebensraum einwirken läßt.

8. Isoxazol-4-yl-benzoylderivate der Formel I gemäß Anspruch 1, in der Z ein 5- oder 6-gliedriger Heteroaromat bedeutet, enthaltend ein bis drei Heteroatome, ausgewählt aus der Gruppe Sauerstoff, Schwefel oder Stickstoff, der gegebenenfalls durch Halogen, Cyano, Nitro, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl substituiert ist oder ein gegebenenfalls durch Halogen, Cyano, Nitro, C₁-C₄-Alkyl oder C₁-C₄-Halogenalkyl substituierter benzokondensierter 5- oder 6-Ring-Heteroaromat;
und L, M, J und Q die in Anspruch 1 genannte Bedeutung haben.

## Claims

1. An isoxazol-4-ylbenzoyl derivative of the formula I 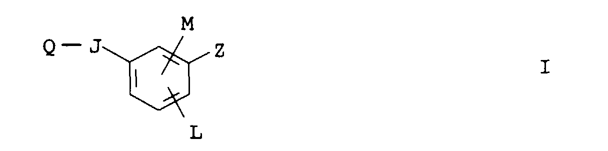 where the substituents have the following meanings:
L and M are hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy, or are halogen, cyano, nitro, a group -(Y)ₙ-S(O)ₘR⁷ or a group -(Y)ₙ-CO-R^{8,}
Z is a 5- or 6-membered heterocyclic saturated or unsaturated radical which has one to three heteroatoms selected from the group consisting of oxygen, sulfur or nitrogen and which is unsubstituted or substituted by halogen, cyano, nitro, a group -CO-R⁸, C₁-C₄-alkyl, C₁-C₄-haloalkyl, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, phenyl which is unsubstituted or substituted by halogen, cyano, nitro, C₁-C₄-alkyl or C₁-C₄-haloalkyl, or an oxo group which may also exist as a hydroxyl group in the tautomeric form, or which forms a bicyclic system together with a fused phenyl ring which is unsubstituted or substituted by halogen, cyano, nitro, C₁-C₄-alkyl or C₁-C₄-haloalkyl, a fused carbocycle or a fused second heterocycle which is unsubstituted or substituted by halogen, cyano, nitro, C₁-C₄-alkyl, di-C₁-C₄-alkylamino, C₁-C₄-alkoxy, C₁-C₄-haloalkoxy or C₁-C₄-haloalkyl,
Y is O or NR^{9,}
n is zero or one,
m is zero, one or two,
R⁷ is C₁-C₄-alkyl, C₁-C₄-haloalkyl or NR⁹R¹⁰,
R⁸ C₁-C₄-alkyl, C₁-C₄-haloalkyl, C₁-C₄-alkoxy or NR⁹R¹⁰,
R⁹ is hydrogen or C₁-C₄-alkyl,
R¹⁰ is C₁-C₄-alkyl,
Q is an isoxazole ring which is linked in the 4-position, of the formula II 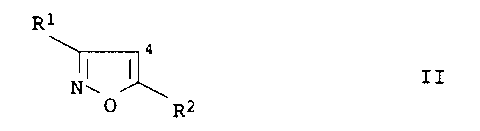
where
R¹ is hydrogen or -CO₂R³,
R² is hydrogen, C₁-C₄-alkyl or C₃-C₆-cycloalkyl, which can be unsubstituted or substituted by one or more halogen atoms or by C₁-C₄-alkyl and
R³ is C₁-C₄-alkyl,
J is a carbonyl radical CO or a radical -CHR⁴; and
R⁴ is an OH group, a hydroxyl group which may be acylated by C₂-C₄-acyl, or a chlorine atom;
or an agriculturally customary salt of a compound I.

2. An isoxazol-4-ylbenzoyl derivative of the formula Ia as claimed in claim 1, 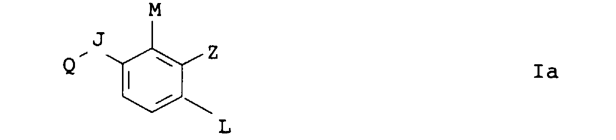 where L is C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-haloalkoxy, C₁-C₄-haloalkylthio, C₁-C₄-alkylsulfonyl, halogen, nitro or cyano and M is hydrogen, C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-haloalkoxy, C₁-C₄-haloalkylthio, C₁-C₄-alkylsulfonyl, halogen, nitro or cyano, and Q, J and Z have the meanings given in claim 1.

3. An isoxazol-4-ylbenzoyl derivate of the formula Ib as claimed in claim 1, 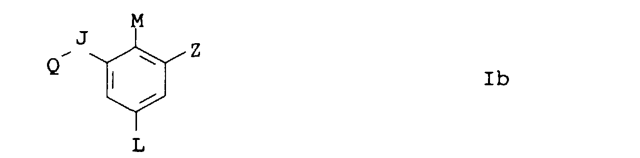 where L and M are C₁-C₆-alkyl, C₂-C₆-alkenyl, C₂-C₆-alkynyl, C₁-C₄-alkoxy, C₁-C₄-alkylthio, C₁-C₄-haloalkyl, C₁-C₄-haloalkoxy, C₁-C₄-haloalkylthio, C₁-C₄-alkylsulfonyl, halogen, nitro or cyano, and Q, J and Z have the meanings given in claim 1.

4. An isoxazol-4-ylbenzoyl derivative of the formula I as claimed in claim 1, where the radicals L or M are hydrogen, methyl, methoxy, methylthio, chlorine, cyano, methylsulfonyl, nitro or trifluoromethyl.

5. A process for the preparation of the isoxazol-4-ylbenzoyl derivatives of the formula I as claimed in claim 1, where Q is a radical CO-J, which comprises reacting alkynyl stannates of the formula A2
(C₄H₉)Sn - C ≡ C- R² A2
with a benzoic acid derivative of the formula III 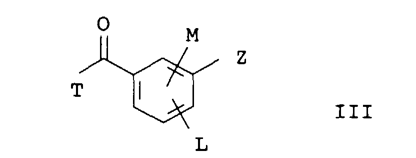 where T is halogen and L, M and Z have the meanings given in claim 1, to give benzoylalkynes of the formula B2 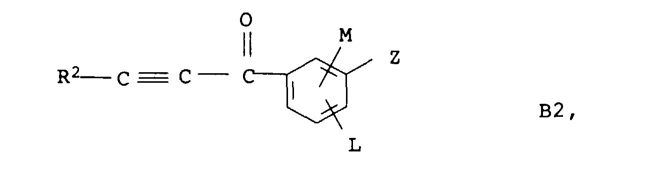 and subjecting these benzoylalkynes together with nitrile oxides of the formula C2 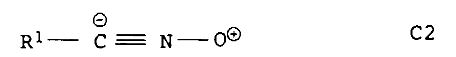 where R¹ is C₁-C₄-alkyl to a cycloaddition reaction to give the isoxazoles of the formula Ic

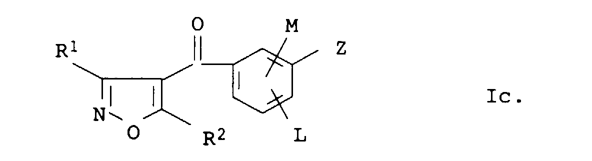

6. A herbicidal composition comprising at least one benzoyl derivative of the formula I as claimed in claim 1 and customary inert additives.

7. A method of controlling undesirable vegetation, which comprises allowing a herbicidally active amount of a benzoyl derivative of the formula I as claimed in claim 1 to act on the plants or their environment.

8. An isoxazol-4-ylbenzoyl derivative of the formula I as claimed in claim 1, where Z is a 5- or 6-membered heteroaromatic ring which has one to three hetero atoms selected from the group consisting of oxygen, sulfur or nitrogen and which is unsubstituted or substituted by halogen, cyano, nitro, C₁-C₄-alkyl, C₁-C₄-haloalkyl, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino or phenyl which is unsubstituted or substituted by halogen, cyano, nitro, C₁-C₄-alkyl or C₁-C₄-haloalkyl, or is a benzo-fused five- or six-membered heteroaromatic ring which is unsubstituted or substituted by halogen, cyano, nitro, C₁-C₄-alkyl or C₁-C₄-haloalkyl;
and L, M, J and Q have the meanings given in claim 1.

## Revendications

1. Dérivés isoxazol-4-yl-benzoyliques de formule I 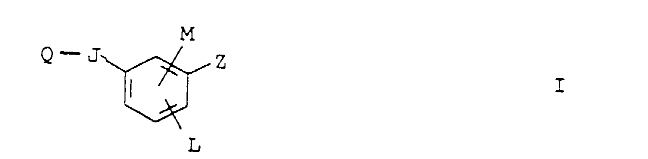 dans laquelle les symboles ont les significations suivantes :
L, M : l'hydrogène, un groupe alkyle en C1-C6, alcényle en C2-C6, alcynyle en C2-C6, alcoxy en C1-C4, ces groupes pouvant porter le cas échéant un à cinq substituants halogéno ou alcoxy en C1-C4 ; un atome d'halogène, un groupe cyano, nitro, un groupe -(Y)ₙ-s(O)ₘR⁷ ou un groupe -(Y)ₙ-CO-R⁸,
Z : un radical hétérocyclique saturé ou insaturé à cinq ou six chaînons contenant un à trois hétéroatomes choisis parmi l'oxygène, le soufre et l'azote, et qui est éventuellement substitué par des halogènes, des groupes cyano, nitro, un groupe -CO-R⁸, alkyle en C1-C4, halogénoalkyle en C1-C4, cycloalkyle en C3-C8, alcoxy en C1-C4, halogénoalcoxy en C1-C4, alkylthio en C1-C4, halogénoalkylthio en C1-C4, di-(alkyle en C1-C4)amino, un groupe phényle éventuellement substitué par des halogènes, des groupes cyano, nitro, alkyle en C1-C4 ou halogénoalkyle en C1-C4, ou un groupe oxo qui peut le cas échéant être sous la forme tautomère de groupe hydroxy, ou qui forme un système bicyclique avec un cycle phényle condensé, éventuellement substitué par des halogènes, des groupes cyano, nitro, alkyle en C1-C4 ou halogénoalkyle en C1-C4, un carbocycle condensé ou un deuxième hétérocycle condensé et éventuellement substitué par des halogènes, des groupes cyano, nitro, alkyle en C1-C4, di-(alkyle en C1-C4)amino, alcoxy en C1-C4, halogénoalcoxy en C1-C4 ou halogénoalkyle en C1-C4,
Y : O, NR⁹
n : 0 ou 1
m : 0, 1 ou 2
R⁷: un groupe alkyle en C1-C4, halogénoalkyle en C1-C4 ou NR⁹R¹⁰,
R⁸: un groupe alkyle en C1-C4, halogénoalkyle en C1-C4, alcoxy en C1-C4 ou NR⁹R¹⁰,
R⁹: l'hydrogène ou un groupe alkyle en C1-C4
R¹⁰ : un groupe alkyle en C1-C4
Q : un cycle isoxazole relié en position 4 et répondant à la formule II 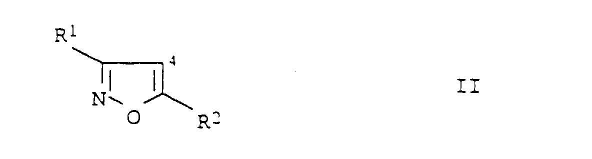
dans laquelle
R¹ représente l'hydrogène ou un groupe -CO₂R³,
R² représente l'hydrogène, un groupe alkyle en C1-C4 ou cycloalkyle en C3-C6 qui peut le cas échéant être substitué par un ou plusieurs atomes d'halogènes ou groupes alkyle en C1-C4 et
R³ représente un groupe alkyle en C1-C4 ;
J représente un groupe carbonyle CO ou un groupe -CHR⁴ ;
R⁴ représente un groupe OH, un groupe hydroxy acylé éventuellement par un groupe acyle en C2-C4 ou un atome de chlore ;
ainsi que les sels des composés I convenant pour les applications agricoles habituelles.

2. Dérivés isoxazol-4-yl-benzéniques de formule Ia selon la revendication 1, 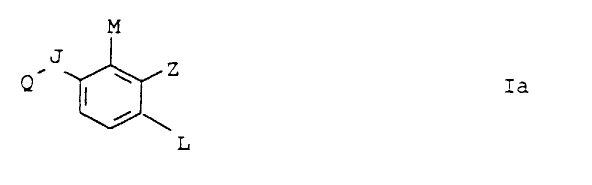 dans laquelle L représente un groupe alkyle en C1-C6, alcényle en C2-C6, alcynyle en C2-C6, alcoxy en C1-C4, alkylthio en C1-C4, halogénoalkyle en C1-C4, halogénoalcoxy en C1-C4, halogénoalkylthio en C1-C4, alkylsulfonyle en C1-C4, un halogène, un groupe nitro ou cyano et M représente l'hydrogène, un groupe alkyle en C1-C6, alcényle en C2-C6, alcynyle en C2-C6, alcoxy en C1-C4, alkylthio en C1-C4, halogénoalkyle en C1-C4, halogénoalcoxy en C1-C4, halogénoalkylthio en C1-C4, alkylsulfonyle en C1-C4, un halogène, un groupe nitro ou cyano et Q, J et Z ont les significations indiquées dans la revendication 1.

3. Dérivés isoxazol-4-yl-benzoyliques de formule Ib selon la revendication 1, 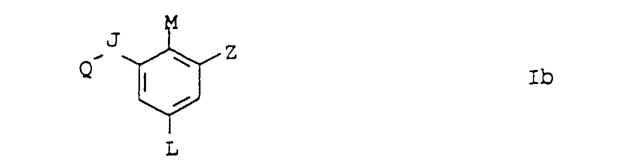 dans laquelle L et M représentent des groupes alkyle en C1-C6, alcényle en C2-C6, alcynyle en C2-C6, alcoxy en C1-C4, alkylthio en C1-C4, halogénoalkyle en C1-C4, halogénoalcoxy en C1-C4, halogénoalkylthio en C1-C4, alkylsulfonyle en C1-C4, des halogènes, des groupes nitro ou cyano et Q, J et Z ont les significations indiquées dans la revendication 1.

4. Dérivés isoxazol-4-yl-benzoyliques de formule I selon la revendication 1 dans laquelle L et M représentent respectivement l'hydrogène, des groupes méthyle, méthoxy, méthylthio, le chlore, des groupes cyano, méthylsulfonyle, nitro ou trifluorométhyle.

5. Procédé de préparation des dérivés isoxazol-4-yl-benzoyliques de formule I selon la revendication 1, dans laquelle Q représente un groupe CO-J, caractérisé par le fait que l'on fait réagir des alcynylstannates de formule A2
(C₄H₉)Sn- C ≡ C - R² A2
avec un dérivé de l'acide benzoïque répondant à la formule III 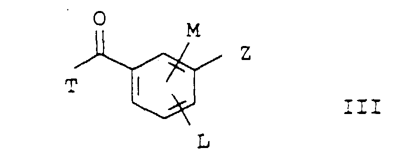 dans laquelle T représente un halogène et L, M et Z ont les significations indiquées dans la revendication 1, ce qui donne des benzolalcynes de formule B2 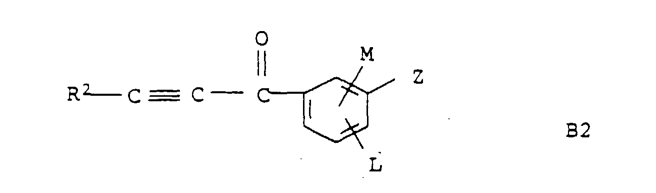 qu'on soumet à une cycloaddition avec des oxydes de nitriles de formule C2 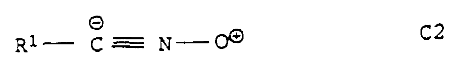 dans laquelle R¹ représente un groupe alkyle en C1-C4, ce qui donne les isoxazoles de formule Ic

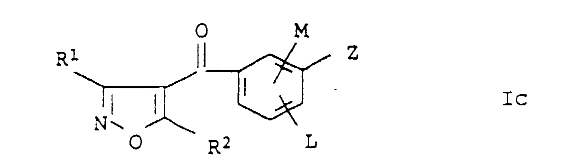

6. Produit herbicide contenant au moins un dérivé benzoylique de formule I selon la revendication 1 et des additifs inertes usuels.

7. Procédé pour combattre les croissances de végétaux indésirables, caractérisé par le fait que l'on fait agir sur les végétaux ou leur habitat une quantité herbicide efficace d'un dérivé benzoylique de formule I de la revendication 1.

8. Dérivés isoxazol-4-yl-benzoyliques de formule I de la revendication 1, dans laquelle Z représente un radical hétéroaromatique à cinq ou six chaînons contenant un à trois hétéroatomes choisis parmi l'oxygène, le soufre ou l'azote, qui est le cas échéant substitué par des halogènes, des groupes cyano, nitro, alkyle en C1-C4, halogénoalkyle en C1-C4, cycloalkyle en C3-C8, alcoxy en C1-C4, halogénoalcoxy en C1-C4, alkylthio en C1-C4, halogénoalkylthio en C1-C4, di-(alkyle en C1-C4)amino, phényle lui-même éventuellement substitué par des halogènes, des groupes cyano, nitro, alkyle en C1-C4 ou halogénoalkyle en C1-C4, ou un radical hétéroaromatique à cinq ou six chaînons benzocondensé et éventuellement substitué par des halogènes, des groupes cyano, nitro, alkyle en C1-C4 ou halogénoalkyle en C1-C4 ;
et L, M, J et Q ont les significations indiquées dans la revendication 1.
